# EUROPEAN PATENT SPECIFICATION

(11) **EP 4 593 865 B1**
(45) Date of publication and mention of the grant of the patent: **06.05.2026**
(21) Application number: 23782935.3
(22) Date of filing: 29.09.2023
(51) Int. Cl.: A61K 38/17, A61P 35/00, C07K 14/00, A61K 47/18

(54) **PHARMACEUTICAL COMPOSITION COMPRISING NFL-TBS40-63 PEPTIDE, VARIANTS OR SALTS THEREOF AND ALANINE**
PHARMAZEUTISCHE ZUSAMMENSETZUNG MIT NFL-TBS40-63 PEPTID, VARIANTEN ODER SALZEN DAVON UND ALANIN
COMPOSITION PHARMACEUTIQUE COMPRENANT UN PEPTIDE NFL-TBS40-63, SES VARIANTS OU SELS ET DE L'ALANINE

(30) Priority: 30.09.2022 EP 22306460
(43) Date of publication of application: 06.08.2025
(73) Proprietor: Gliocure, 49933 Angers Cedex 9 (FR)
(72) Inventor: BALDWIN, Paul, 49100 ANGERS (FR); BOUCHET, Benjamin, 72210 CHEMIRE LE GAUDIN (FR)
(74) Representative: Regimbeau
(86) International application number: PCT/EP2023/077179
(87) International publication number: WO 2024/068999

(56) References cited:
- EP-A1- 2 332 560
- CARRADORI DARIO ET AL: "NFL-lipid nanocapsules for brain neural stem cell targetingin vitroandin vivo", JOURNAL OF CONTROLLED RELEASE, ELSEVIER, AMSTERDAM, NL, vol. 238, 5 August 2016 (2016-08-05), pages 253 - 262, XP029712439, ISSN: 0168-3659, DOI: 10.1016/J.JCONREL.2016.08.006
- BOCQUET A ET AL: "Neurofilaments Bind Tubulin and Modulate Its Polymerisation", THE JOURNAL OF NEUROSCIENCE, SOCIETY FOR NEUROSCIENCE, US, vol. 29, no. 35, 2 September 2009 (2009-09-02), pages 11043 - 11054, XP002581714, ISSN: 0270-6474
- LÉPINOUX-CHAMBAUD CLAIRE ET AL: "The Neurofilament-Derived Peptide NFL-TBS.40-63 Targets Neural Stem Cells and Affects Their Properties", vol. 5, no. 7, 13 May 2016 (2016-05-13), US, pages 901 - 913, XP093032042, ISSN: 2157-6564, Retrieved from the Internet <URL:https://api.wiley.com/onlinelibrary/tdm/v1/articles/10.5966%2Fsctm.2015-0221> DOI: 10.5966/sctm.2015-0221

## Description

### TECHNICAL FIELD OF THE INVENTION

The present invention relates to a pharmaceutical composition comprising NFL-TBS₄₀₋₆₃ peptide, biologically active variants or pharmaceutically acceptable salts thereof and alanine in specific concentrations. The present invention also relates to a manufacturing method of said composition and to the application of this composition as a medicament, particularly for treating cancer.

### BACKGROUND OF THE INVENTION

Cell division, or mitosis, is the process that enables cells to multiply in order to construct or regenerate tissues or to replace dead cells. In cancer cells the regulation of this process is faulty, which is why these cells divide uncontrollably and thus create tumors. One effective therapeutic route for combating cancers comprises blocking the division of cancer cells.

Tubulin is a fundamental molecule in the process of cell division. In fact, it polymerizes to form the microtubules that are indispensable for intracellular transport during mitosis.

It was found in the past that fragments of intermediate filaments (from neurofilaments) can bind tubulin and alter microtubule polymerization, thus blocking cell division. These fragments (peptides) are disclosed in application WO 2005/121172 as corresponding to the tubulin-binding site (TBS), located in intermediate filament proteins (namely the neurofilament light chain protein NFL, keratin 8, GFAP, and vimentin). In addition, international application WO 2011/073207 and Bocquet *et al.* (2009) disclosed that the peptide sequence of the second tubulin-binding site of the NFL protein (called "NFL-TBS.₄₀₋₆₃") is able to inhibit the proliferation of neuroblastoma and glioblastoma cell lines ***in vitro.***

The efficiency of NFL-TBS.₄₀₋₆₃ and its biologically active derivatives in the treatment of cancer, and particularly of glioblastoma was previously demonstrated. This peptide thus appears as a promising treatment of cancer.

To be used as a medicament, NFL-TBS.₄₀₋₆₃ peptide has to be integrated in a pharmaceutical composition.

Given that this peptide cannot be administered via the oral route (since it is degraded in the digestive tract), the parenteral route is the preferred route.
The simplest parenteral formulation for any drug is a true, isotonic, aqueous solution, ideally buffered at a set pH. However, formulation of a parenterally acceptable buffered, isotonic, true aqueous solution of NFL-TBS.₄₀₋₆₃ peptide proved to be a substantial challenge.

Firstly, NFL-TBS.₄₀₋₆₃ peptide has a low intrinsic aqueous solubility (< 2.5 mg/mL), which is practically unaffected by nearly all parenterally acceptable co-solvents and surfactants at pharmaceutically acceptable concentrations.

Secondly, NFL-TBS.₄₀₋₆₃ peptide is insoluble in nearly all commonly used parenteral buffer systems and tends to irreversibly precipitate in the presence of sodium or potassium ions (which are present in most buffer systems).

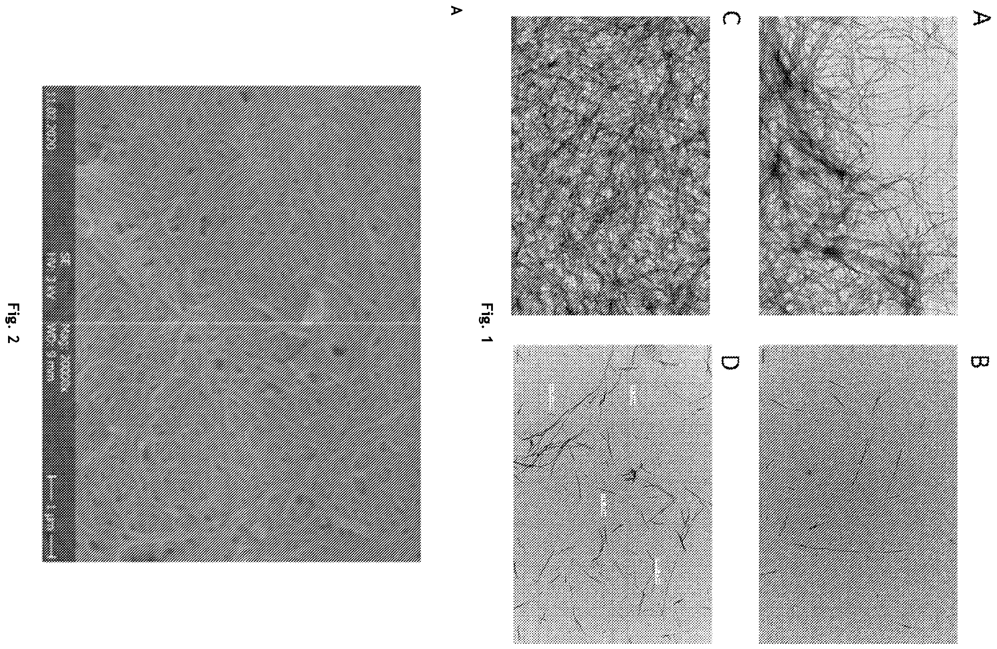

Finally, and most importantly, NFL-TBS.₄₀₋₆₃ peptide self-associates to form sub-visible fibrillar structures (up to approximately 2 µm in length). Fibril structures are highly undesirable in an injectable solution due to the risk that they could cause unwanted side effects. Furthermore, their presence hampers industrial production of a viable, sterile drug product: it was found that solutions of NFL-TBS.₄₀₋₆₃ peptide at 1mg/mL in water contain large, tangled masses of fibrils that very quickly block a 0.2µm sterilizing filter (**Figure 1**). In addition, given that peptides are highly heat sensitive, peptide solutions cannot be sterilized by autoclaving. Furthermore, gamma irradiation of aqueous solutions is not advised due to formation of free radicals which can extensively degrade drug products. Aseptic processing carries an inherent risk of non-sterility and is not a preferred production method (EMA guideline: EMA/CHMP/CVMP/QWP/850374/201). Furthermore, aseptic processing would not resolve the problem of the fibrils being present in the formulation.

From the above, it appears that there are several problems (low solubilization, formation of fibrils, difficult sterilization) to be solved in order to obtain a pharmaceutical composition, wherein the therapeutic activity of the peptide is not decreased, and which is appropriate for parenteral administration. In addition, this pharmaceutical composition has to be suitable for manufacturing in industrial conditions and has to remain stable over time.

Golonov *et al.* (2004) discloses that the solubility of proteins and peptides can be increased by the use of 50mM glutamine and 50mM arginine. However, this combination of amino acids did not increase the solubility of NFL-TBS.₄₀₋₆₃ peptide, and in fact it gave a lower solubility as compared to the intrinsic solubility of NFL-TBS.₄₀₋₆₃ peptide in water (<0.8mg/mL compared to < 2.5 mg/mL, respectively).

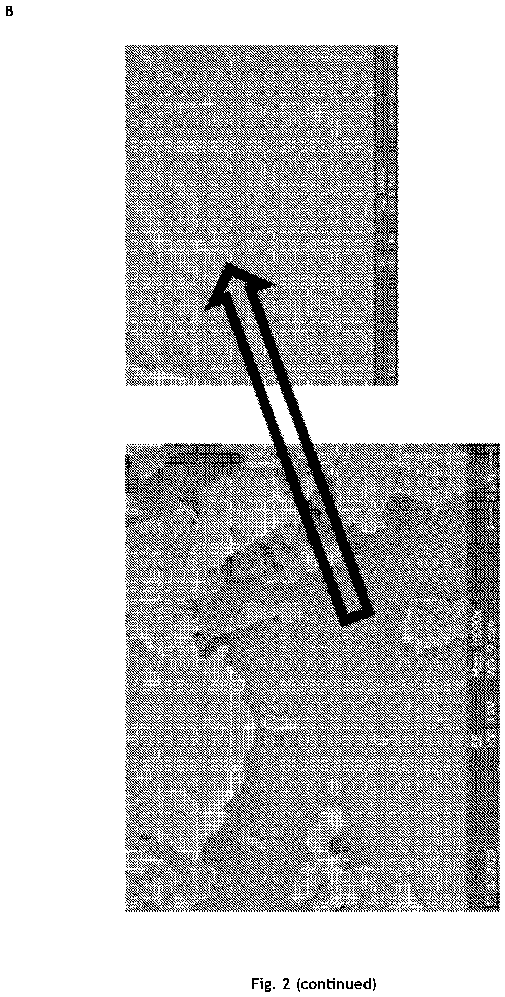

Maggio *et al.* (2010) and Hamada *et al.* (2009) discloses various excipients which are used to prevent peptide aggregation and fibril formation, but these excipients did not improve the solubility of NFL-TBS.₄₀₋₆₃ peptide. In fact, much of the literature in this field focuses on the formation of fibrils in pharmaceutical peptide/proteins over time and/or on agitation, and much of the research in this field is aimed at slowing or eliminating the formation of fibrils (i.e. they are not present initially, and excipients are used to slow/stop their formation). In contrast, it was found that the NFL-TBS.₄₀₋₆₃ peptide drug substance powder used for therapeutical purpose already contains fibrils (Figure 2), which are released into the liquid medium when the drug substance is added to liquid. Thus, with NFL-TBS.₄₀₋₆₃ peptide the key issue was to decrease the number and the size of the released fibrils, and preferably to eliminate them, via dissolving, rather than preventing their formation over time.

Therefore, there is need to provide a pharmaceutical composition, wherein NFL-TBS.₄₀₋₆₃ peptide is dissolved sufficiently, notably by dissolving already existing fibrils, in order to allow sterilization by filtration and the manufacturing of an injectable composition for parenteral administration. In addition, this composition has to be suitable for manufacturing in industrial conditions and to be stable over the time.

### SUMMURY OF THE INVENTION

When preparing a pharmaceutical composition comprising NFL-TBS.₄₀₋₆₃ peptide, biologically active variants or pharmaceutically acceptable salts thereof for a parenteral administration, the inventors surprisingly found that the addition of alanine in a specific concentration range allows to significantly improve the solubility of this peptide and its variants and salts, by reducing the size and the quantity of fibrils present in the composition and even by completely eliminating these fibrils. Such a pharmaceutical composition can thus be easily sterilized by filtration and used for parenteral administration into a patent in need thereof.

Thus, according to first aspect, the present invention relates to a pharmaceutical composition comprising NFL-TBS₄₀₋₆₃ peptide, biologically active variants or pharmaceutically acceptable salts thereof, an alanine and pharmaceutically acceptable excipients, wherein the alanine is at a concentration comprised between 25 mM and 120 mM.

Since this composition is easily sterilizable, it is thus suitable for manufacturing in industrial conditions. This composition may be manufactured in an easy manner following a multistep manufacturing method.

In accordance to a second aspect, the present invention also relates to a method for manufacturing a pharmaceutical composition according to the invention comprising the following steps:
a) preparing a solution, preferably an aqueous solution, comprising a buffer, an osmolarity adjusting agent, one or more solubilizing agent(s) and alanine, preferably L-alanine at a concentration comprised between 25 mM and 120 mM;
b) adding NFL-TBS₄₀₋₆₃ peptide, biologically active variants or pharmaceutically acceptable salts thereof to the solution prepared in step a);
c) pre-filtering the formulation obtained in step b) by using a filter with pore size compromised preferably between 0.3 and 1.0 µM, and
d) sterilizing the pre-filtered formulation of step c) by using a filter with pore size comprised preferably between 0.1 and 0.2 µM.

As described below, further steps may be added to the above method in order to improve the solubility of the peptide and to decrease the size and the number of fibrils until fully eliminating them and thus facilitating the filtration and the sterilization of the final pharmaceutical composition.

As mentioned above, the active principal of the composition of the present invention is NFL-TBS₄₀₋₆₃ peptide, biologically active variants or pharmaceutically acceptable salts thereof. They are able to block the polymerization of tubulin and thus decrease cell division. This peptide may be thus successfully used as a therapeutic agent. The composition of the present invention is adapted to parenteral administration since it allows very good peptide solubilization and sterilization. This composition may be thus used for treating patients in need thereof by parenteral administration.

In accordance with a third aspect, the pharmaceutical composition of the present invention is for use as a medicament, particularly for treating cancer.

### DETAILLED DESCRIPTION

As mentioned above, NFL-TBS₄₀₋₆₃ peptide has very low solubility (< 2.5mg/mL in water), and most of the classical excipients used to increase drug substance solubility were of little or no use.

In addition, a drug product for parenteral administration must be sterile. Terminal sterilization in the final packaging (for example by autoclaving at ≥ 121°C for ≥ 15 minutes) is a preferable technical solution. However, peptides do not support such high temperatures, and hence sterilizing filtration (preferably by 0.1 or 0.2µm filter) is the next best choice for a peptide drug product. The main difficulty which prevents NFL-TBS₄₀₋₆₃ peptide filtration is due to the presence of fibrils which, if not properly dissolved, quickly block sterilizing filters, even if a pre-filter is used. Since a peptide solution product cannot be sterilized by any other method, filter-blocking is a show-stopper for industrial production of a pharmaceutical composition comprising NFL-TBS₄₀₋₆₃ peptide adapted to parenteral administration.

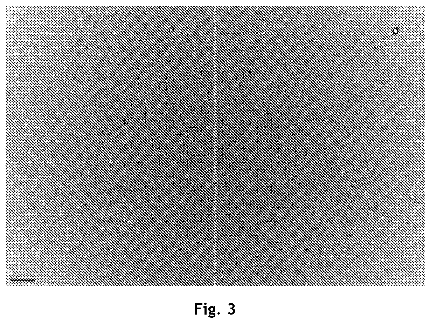

When the inventors were testing different solution compositions for rendering the NFL-TBS₄₀₋₆₃ peptide more soluble (notably by decreasing fibril size and number, and ideally even by eliminating fibrils present in the solution) and thus, sufficiently filtrable to be used in parenteral drug composition, they surprisingly found that a particular amino acid, alanine, in particular concentrations (comprised between 25 and 120 mM) when added in the solution, allowed solubilization of the NFL-TBS₄₀₋₆₃ peptide, and significantly reduced the number and the size of fibrils (**Figure 3**). As far as inventors know, the use of alanine for improving the solubilization of self-assembling peptides and particularly of NFL-TBS₄₀₋₆₃ peptide has never been disclosed in the relating art. Furthermore, the inventors surprisingly found that the solubilizing effect of the alanine was only present with specific concentrations of alanine (comprised between 25 mM and 120 mM) and that the solubilizing effect was lost at higher or lower concentrations of alanine.

According to one aspect, the present invention thus relates to a pharmaceutical composition comprising NFL-TBS₄₀₋₆₃ peptide, biologically active variants or pharmaceutically acceptable salts thereof, an alanine and pharmaceutically acceptable excipients, wherein the alanine is at concentrations comprised between 25 mM and 120 mM.

In the context of the present application, the terms **"composition"** and **"formulation"** are used interchangeably to designate a liquid medium, preferably an aqueous medium containing different chemical agents appropriate for pharmaceutical use for parenteral administration, alanine and NFL-TBS₄₀₋₆₃ peptide, biologically active variants or pharmaceutically acceptable salts thereof. The term **"solution"** is used herein to designate a liquid medium, preferably an aqueous medium containing different chemical agents that are dissolved in the medium and are appropriate for pharmaceutical use for parenteral administration, and alanine (i.e. without the peptide, biologically active variants or pharmaceutically acceptable salts thereof).

Regarding NFL-TBS₄₀₋₆₃ peptide, as mentioned above, this compound has been already disclosed in the application WO2011/073207 (having a sequence identified as SEQ ID NO: 1). NFL-TBS₄₀₋₆₃ peptide has a sequence consisting in 24 amino acids (the amino acid sequence also disclosed in Berges *et al.,* 2012). As mentioned previously, it corresponds to the second tubulin-binding site of the light neurofilament subunit (amino acids 40 to 63 of the TBS site of the NFL protein).

According to one embodiment, the sequence of NFL-TBS₄₀₋₆₃ peptide bears a modification at N- and/or C-terminals, particularly, modifications such as biotin, CONH₂ phosphorylation, acetylation etc. According to preferred embodiment, the sequence of NFL-TBS₄₀₋₆₃ peptide bears a biotin molecule at N-terminal and CONH₂ group (amide group) at C-terminal (indicated in the examples as GC01 and/or Biot-NFL).

NFL-TBS₄₀₋₆₃ peptide included in the composition of the present invention has two predicted conformations: β-sheet and α-helix (disclosed in Berges *et al.,* 2012).

The pharmaceutical composition of the present invention may comprise biologically active variants of NFL-TBS₄₀₋₆₃ peptide. In the context of the application, the term **"biologically active variants of NFL-TBS₄₀₋₆₃ peptide"** relates to variants of this peptide retaining the biological activity and the specificity of the parent NFL-TBS₄₀₋₆₃ peptide. Thus, in the context of the invention, the "biologically active" variants of the NFL-TBS₄₀₋₆₃ peptide have to show a high biological capacity for inhibiting the tubulin polymerization and thus must possess a strong anti-proliferative effect. Preferably, the antiproliferative effect of the NFL-TBS₄₀₋₆₃ peptide variants on cancer cells for example has to be of at least about 70%, preferably above 80%, more preferably above 90% and even more preferably above or equal to 100% of the antiproliferative effect of the parent NFL-TBS₄₀₋₆₃ peptide, as assessed *in vitro* by conventional proliferation techniques. The variants of the peptide can be a peptidomimetic variants, which is an organic molecule that mimics some properties of the parent peptide, including at least one or more properties of interest that preferably is its biological activity. Preferred peptidomimetics are obtained by structural modification NFL-TBS₄₀₋₆₃ peptide, preferably using unnatural amino acids, such as D amino acids instead of L amino acids, conformational restraints, isosteric replacement, cyclization, or other modifications. Other preferred modifications include, without limitation, those in which one or more amide bonds are replaced by a non-amide bond, and/or one or more amino acid side chain is replaced by a different chemical moiety, or one of more of the N-terminus, the C-terminus or one or more side chain is protected by a protecting group, and/or double bonds and/or cyclization and/or stereospecificity is introduced into the amino chain to increase rigidity and/or binding affinity. Still other preferred modifications include those intended to enhance resistance to enzymatic degradation, improvement in the bioavailability, and more generally in the pharmacokinetic properties, compared to the parent NFL-TBS₄₀₋₆₃ peptide. All of these variations are well known in the art. Thus, given the peptide sequences of the NFL-TBS₄₀₋₆₃ peptide, those skilled in the art are enabled to design and produce peptidomimetics having biological characteristics similar to or superior to such peptides. Preferred peptidomimetic variants have the same amino acid sequence as the NFL-TBS₄₀₋₆₃ peptide comprising a chemical modification at N- and/or C-terminal, the final peptide retaining the biological activity of the parent NFL-TBS₄₀₋₆₃ peptide.

According to one embodiment, NFL-TBS₄₀₋₆₃ peptide, biologically active variants or pharmaceutically acceptable salts thereof are present in the pharmaceutical composition of the invention at a concentration comprised between 1 to 20 mg/ml, preferably between 2 to 15 mg/ml more preferably between 3 to 10 mg/mL and even more preferably is 3 mg/ml.

The pharmaceutical composition of the invention may also comprise a pharmaceutically acceptable salt of NFL-TBS₄₀₋₆₃ peptide. As used herein, the term **"pharmaceutically acceptable salt of NFL-TBS₄₀₋₆₃ peptide"** relates to salts which are appropriate to be used in pharmaceutical compositions, particularly adapted to parenteral administration. According to one embodiment, a pharmaceutical acceptable salt for NFL-TBS₄₀₋₆₃ peptide is selected from the group of salts comprising, hydrochloride, sodium, sulphate, acetate, phosphate or diphosphate, chloride, potassium, maleate, calcium, citrate, mesylate, nitrate, tartrate, aluminium, gluconate etc. According to preferred embodiment, the pharmaceutical acceptable salt of NFL-TBS₄₀₋₆₃ peptide is an acetate salt.

As mentioned previously, the inventors surprisingly found that a specific concentration of alanine in the composition of the invention allows to significantly improve the solubilization of NFL-TBS₄₀₋₆₃ peptide, its variants and salts.

As used herein, the term **"alanine"** relates to an α-amino acid that is used in the biosynthesis of proteins. It contains an amine group and a carboxylic acid group, both attached to the central carbon atom which also carries a methyl group side chain. Its IUPAC systematic name is 2-aminopropanoic acid, and it is classified as a nonpolar, aliphatic α-amino acid. It is encoded by all codons starting with GC (GCU, GCC, GCA, and GCG). Its chemical formula is C₃H₇NO₂.

According to the preferred embodiment, alanine comprised in the pharmaceutical composition of the invention is L-alanine (CAS # 56-41-7) which is the L-isomer of alanine and has the following structural chemical formula:

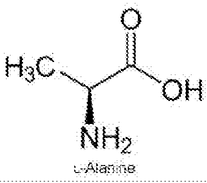

The inventors demonstrated that alanine, preferably L-alanine is able to improve the solubilization of NFL-TBS₄₀₋₆₃ peptide added to a pharmaceutical composition, if alanine, preferably L-alanine is present in the composition at concentration comprised between 25 and 120 mM. Below or above this range, alanine, preferably L-alanine has no beneficial effect on the solubility of said peptide, nor on the solubilization of its fibrils. Indeed, the inventors demonstrated that with no alanine, and at high concentration (225 mM) of alanine, the solubilization of said peptide is decreased (corresponding to grade 3 of the examples). Preferably, the concentration of alanine, preferably L-alanine is comprised between 25 and 120 mM, more preferably between 40 and 70 mM and even more preferably, between 40 and 60 mM, even more preferably is 50 mM.

The composition of the present invention also comprises pharmaceutically acceptable excipients other than alanine. In the sense of the present invention, **"pharmaceutically acceptable excipient"** means any material that is suitable for use in a pharmaceutical product. Particularly, these excipients are selected from the group consisting of a buffer, an osmolarity adjusting agent and one or more traditional solubilizing agent(s).

The buffer is a pharmaceutically acceptable buffer appropriate to parenteral administration. The preferable buffer is selected from the group consisting of phosphate, citrate, acetate, lactic acid, tromethamine, gluconic acid, tartaric acid, succinate, malic, fumaric, α-ketoglutaric, or an amino acid which is naturally positively charged at physiological pH, (particularly, slightly positively charged at physiological pH) such as a histidine. More preferably, the buffer is L-histidine. The concentration of the buffer in the composition of the invention is comprised in the range between 10 and 100 mM, preferably between 15 and 70 mM, more preferably, between 20 and 50 mM and even more preferably between 25 and 30 mM.

The osmolarity adjusting agent in the composition of the invention is an agent which is able to adjust the osmotic pressure so that it corresponds to the physiological one. Preferably, the osmolarity adjusting agent is a polyol compound, more preferably a glycerol. The composition according to the invention comprises preferably 0.5% to 5%, more preferably 1% to 3% and even more preferably 1% or 2% of said agent of the total concentration of the composition, so that it is iso-tonic (ca. 300 ± 20 mOmol/Kg) in combination with the other components of the composition.

Concerning the solubilizing agent, this agent may be selected from the group consisting of a cyclodextrin, polypropylene glycol and polyethylene glycol (PEG). Particularly, the concentration of the solubilizing agent is comprised between 0.5% and 50%, more particularly between 1% and 20%, even more particularly, between 5% and 10% of the total concentration of the composition.

Cyclodextrin used in the composition according to the invention is selected from the group consisting of hydroxypropyl-α-cyclodextrin (HPαCD); hydroxypropyl-β-cyclodextrin (HPBCD); dimethy-β-cyclodextrin (DMBCD), sulphobutylether-β-cyclodextrin (SBEBCD), randomly methylated-β-cyclodextrin (RMBCD), hydroxypropyl-γ-cyclodextrin (HPγCD) and 2-hydroxypropyl-β-cyclodextrin (2-HP-β-CD). Preferably, hydroxypropyl-β-cyclodextrin (2-HP-B-CD) is used as solubilizing agent.

Polyethylene glycol (PEG) is selected from any one of PEG200, PEG300 and PEG400. The various excipients of the solution used for solubilizing NFL-TBS₄₀₋₆₃ peptide, its variants and pharmaceutically acceptable salts may be combined in several manners by selecting any one of pharmaceutical acceptable buffers cited above at any one of the concentrations comprised in the concentration ranges, to be combined with any one of the cited osmolarity adjusting agents and solubilizing agents at any one of the concentrations cited in the above concentration ranges. To this combination of pharmaceutical expedients may be added various concentrations of alanine, preferably L-alanine selected from those cited in the corresponding range above. Finally, different concentrations of NFL-TBS₄₀₋₆₃ peptide, its variants and pharmaceutically acceptable salts from those cited in the above corresponding range, may be added to any one of the various combinations of pharmaceutical excipients and alanine.

According to one embodiment, the pharmaceutical composition of the present invention is a liquid solution, preferably an aqueous solution comprising:
- NFL-TBS₄₀₋₆₃ peptide, biologically active variants or pharmaceutically acceptable salts thereof at a concentration comprised between preferably between 1 and 20 mg/ml more preferably between 3 and 10 mg/ml and even more preferably is 3 mg/ml
- an alanine at concentration comprised between 25 and 120 mM, more preferably between 40 and 70 mM and even more preferably, between 40 and 60 mM.,
- L-histidine at concentration comprised between 10 and 100 mM, preferably between 15 and 70 mM, more preferably, between 20 and 50 mM and even more preferably between 25 and 30 mM,
- a glycerol, preferably at 0.5% to 5%, more preferably at 1% to 3% and even more preferably 1% to 2% of the total concentration of the composition and
- a cyclodextrin, preferably 2-Hydroxypropyl-β-cyclodextrin or sulfobuytlether- β-cyclodextrin at concentration comprised between 0.5% and 50%, more particularly 1% and 20%, even more particularly, between 5% and 10% of the total concentration of the composition.

According to a preferred embodiment, the pharmaceutical composition of the present invention is an aqueous solution comprising:
- NFL-TBS₄₀₋₆₃ peptide at a concentration of 3 mg/ml;
- a L-alanine at concentration comprised between 50 and 60 mM, preferably 50 mM.,
- L-histidine at concentration between 25 and 30 mM, preferably 25 mM,
- a glycerol, at 1% to 2% of the total concentration of the composition and- 2-Hydroxypropyl-β-cyclodextrin at concentration comprised between 5% and 10%, preferably 10% of the total concentration of the composition.

As indicated above, the aim of the present invention is also to provide a pharmaceutically acceptable composition, adapted to parenteral administration, which can be manufactured easily by an industrial manufacturing method, and which remains stable over time.

According to the second aspect, the present invention thus relates to a method for manufacturing a pharmaceutical composition according to the invention comprising the following steps:
a) preparing a solution, preferably an aqueous solution, comprising a buffer, an osmolarity adjusting agent, one or more solubilizing agent(s) and alanine, preferably a L-alanine;
b) adding NFL-TBS₄₀₋₆₃ peptide, biologically active variants or pharmaceutically acceptable salts thereof to the solution prepared in step a);
c) pre-filtering the formulation obtained in step b) by using a filter with pore size compromised preferably between 0.3 and 1 µM, and
d) sterilizing the pre-filtered formulation of step c) by using a filter with pore size comprised preferably between 0.1 and 0.2 µM.

According to one embodiment of the method of the invention, in step a):
- the buffer is selected from the group consisting of phosphate, citrate, acetate, lactic acid, tromethamine, gluconic acid, tartaric acid, succinate, malic, fumaric, α-ketoglutaric, an amino acid with positively charged said chain, preferably L-histidine;
- the osmolarity adjusting agent is a polyol compound, preferably a glycerol, and
- the solubilizing agent is selected from the group consisting of a cyclodextrin, polypropylene glycol or polyethylene glycol (PEG).

According to a preferred embodiment of said method, the buffer, the adjusting agent and the solubilizing agent may be selected from those described above at the concentrations selected from the ranges described above and combined in several manners as previously indicated. In addition, alanine and NFL-TBS₄₀₋₆₃ peptide, biologically active variants or pharmaceutically acceptable salts thereof are also used in the ranges of concentration described above.

The inventors of the present invention also found that the solubilization of NFL-TBS₄₀₋₆₃ peptide may be improved by including some additional steps in the manufacturing method and/or by varying specific parameters in a specific manner.

Particularly, the inventors of the present invention found that NFL-TBS₄₀₋₆₃ peptide when it is in the form of powder, may be micronized before adding the peptide to the solubilizing solution, in order to reduce the initial fibril size in the drug substance powder. Thus, according to one embodiment, before adding NFL-TBS₄₀₋₆₃ peptide powder, the drug substance is micronized, preferably by using jet-milling or ball-milling. The micronization allows to break-up some of the fibrils already present in the peptide powder and thus, the solubilization of the peptide in the solubilizing solution and its filtration and sterilization are facilitated. The peptide powder may be also lyophilized.

In addition, the inventors found that heating the solubilizing solution at temperature comprised between 20°C and 70°C, preferably between 30°C and 50°C and more preferably between 40°C and 45°C before and/or after adding NFL-TBS₄₀₋₆₃ peptide, biologically active variants or pharmaceutically acceptable salts thereof, allows to increase the solubility of the fibrils and thus facilitates the sterilizing filtration step.

According to one embodiment, the manufacturing method of the present invention thus comprises a further step of heating the solution obtained in step a) and/or the formulation obtained in step b) at a temperature comprised between 20°C and 70°C, preferably between 40°C and 45°C.

The inventors also found that when NFL-TBS₄₀₋₆₃ peptide, biologically active variants or pharmaceutically acceptable salts thereof is added in step b) under agitation, the solubility of the fibrils is also improved.

According to another embodiment of the manufacturing method of the invention, the addition of NFL-TBS₄₀₋₆₃ peptide, biologically active variants or pharmaceutically acceptable salts thereof in step b) is performed under agitation.

According to preferred embodiment, the agitation is performed via magnetic or paddle stirring at between 1 to 1500 rpm, preferably between 100 to 1000 rpm, more preferably between 300 to 600 rpm.

The time of stirring is comprised between 15 min to 6 hours, preferably, between 1 to 3 hours and more preferably, between 1 to 2 hours. Stirring during this time window allows to significantly decrease the number of fibrils in the peptide solution.

More preferably, the stirring is performed at a speed between 300 to 600 rpm, during 1 to 2 hours at a temperature comprised between 30°C and 50°C, more preferably between 40 to 45°C.

According to a particularly preferred embodiment, the manufacturing method of the invention, comprises heating the buffer solution to 40-45°C prior to addition at step b) of the NFL-TBS₄₀₋₆₃ peptide, biologically active variants or pharmaceutically acceptable salts thereof, then maintaining the composition at the set temperature for 1.5 ± 0.5 hours under agitation at 300 to 600 rpm.

Step c) of the said manufacturing method is a pre-filtration of the formulation obtained in step b) through a filter having a pore size comprised between 0.3 and 1.0 µm, preferably between 0.4 and 0.5µm and more preferably, 0.45µm.

Step d) of sterilization is performed by using a filter having a pore size between 0.1 and 0.2µm, preferably 0.1 or 0.2 µm.

The present invention further relates to a composition obtained by the method disclosed above.

As previously indicated, the pharmaceutical composition according to the present invention is for medical use given that of NFL-TBS₄₀₋₆₃ peptide, biologically active variants or pharmaceutically acceptable salts thereof have therapeutical efficiency.

Thus, in accordance to a third aspect, the pharmaceutical composition of the present invention is used as a medicament.

Particularly, it was demonstrated previously that NFL-TBS₄₀₋₆₃ peptide, biologically active variants or pharmaceutically acceptable salts thereof, alter tubulin polymerization and consequently, cell proliferation is reduced. This anti-proliferative ability of the peptide is successfully used for treating proliferative diseases such as cancer.

According to one embodiment, the pharmaceutical composition of the present invention is thus used as a medicament for treating cancer.

The term **"treatment",** as used here, refers to any action to reduce or eliminate the symptoms or causes of cancer. A treatment in the sense of the invention may include the administration of the pharmaceutical composition or a product containing the same.

As used herein, **"cancer"** refers to a malignant neoplasm characterized by deregulated or uncontrolled cell growth.

The term **"cancer"** includes primary malignant tumours (e.g., those whose cells have not migrated to sites in the subject's body other than the site of the original tumour) and secondary malignant tumours (e.g., those arising from metastasis, the migration of tumour cells to secondary sites that are different from the site of the original tumour). Such cancer may notably be a solid cancer, or a hematopoietic cancer.

According to one embodiment the cancer treated by the pharmaceutical composition of the present invention is a solid peripheral cancer, such as, but not limited to, liver cancer, prostate cancer or skin cancer (as defined in the international application WO 2005/121172) or a cancer of the central nervous system, such as, but not limited to, primary brain cancer, brain metastases arising from solid peripheral cancers, malignant glioma selected from the group consisting of anaplastic astrocytoma (AA), glioblastoma multiform (GBM), anaplastic oligodendroglioma (AO), and anaplastic oligoastrocytoma (AOA) (as defined in the international application WO 2011/073207).

Thus, according to one embodiment, the pharmaceutical composition of the present invention is for use in the treatment of cancer as defined above.

According to the preferred embodiment, the cancer is selected from the group of solid peripheral cancers, primary brain cancers and brain metastasis arising from a solid peripheral cancer. Particularly, the cancer is a glioblastoma.

As indicated through the present application, the aim of the present invention is also to provide a pharmaceutical composition comprising NFL-TBS₄₀₋₆₃ peptide, biologically active variants or pharmaceutically acceptable salts thereof which is adapted to parenteral administration.

Therefore, according to one embodiment, the pharmaceutical composition of the present invention is an injectable composition, or a composition adapted to slow-release delivery (for example by a pump or diffusion gel) for parenteral administration. This injectable composition or the composition adapted to slow-release delivery is used for treating the above-mentioned diseases.

In the context of the present invention, a **"parenteral administration"** relates to a drug administration by a route different from oral administration (enteral administration) and delivery different from delivery to the gastrointestinal tract, nasal cavity, vaginal cavity, the eyes or the ears. The parenteral administration may be performed by the use of systemic injection or indwelling catheter through different routes selected from the epidural, intracerebral, intra-arterial, intra-articular subcutaneous, intravenous, intramuscular or intraperitoneal routes.

According to one embodiment, the pharmaceutical composition of the present invention is administered by the intravenous route.

The pharmaceutical composition of the invention is administered in a therapeutically effective amount.

As used herein, **"a therapeutically effective amount"** means an amount sufficient to influence the therapeutic course of a particular disease condition. A therapeutically effective amount is also the amount at which all toxic or secondary effects of the composition are offset by the therapeutic beneficial effects of the active principle used.

Optimal modes of administration, dosages and dosage forms can be determined according to the criteria generally taken into account in the establishment of a treatment adapted to a patient, such as the patient's age or body weight, the severity of his or her general condition, tolerance to the treatment and the side effects observed.

The dosage naturally depends on the method of administration, the therapeutic indication, the patient's age and condition.

The dose to be administered for a human is preferably from 0.001 to 250 mg/kg body weight (BW) of NFL-TBS₄₀₋₆₃ peptide, biologically active variants or pharmaceutically acceptable salts thereof per day, preferably from 0.01 to 100 mg/kg BW of NFL-TBS₄₀₋₆₃ peptide, biologically active variants or pharmaceutically acceptable salts thereof per day, more preferably from 0.1 to 60 mg/kg BW of NFL-TBS₄₀₋₆₃ peptide, biologically active variants or pharmaceutically acceptable salts thereof per day and even more preferably 1 to 50 mg/kg BW of NFL-TBS₄₀₋₆₃ peptide, biologically active variants or pharmaceutically acceptable salts thereof per day.

The unit dose of NFL-TBS₄₀₋₆₃ peptide, biologically active variants or pharmaceutically acceptable salts thereof preferably includes 0.1 to 60 mg/kg BW of this compound.

According to one embodiment of the present invention, the initial dose of NFL-TBS₄₀₋₆₃ peptide, biologically active variants or pharmaceutically acceptable salts thereof administered may if necessary be adjusted during treatment according to the response of the treated subject to this treatment. Based on this general knowledge and on the present description, the skilled person will be able to adjust the dose of NFL-TBS₄₀₋₆₃ peptide, biologically active variants or pharmaceutically acceptable salts thereof so as to optimize its therapeutic effect.

According to one embodiment, the pharmaceutical composition of the present invention comprises NFL-TBS₄₀₋₆₃ peptide, biologically active variants or pharmaceutically acceptable salts thereof as active principle in a concentration comprised between 0.001 mg/kg to 250 mg/kg BW, preferably 0.01 to 100 mg/kg BW, more preferably 0.1 to 60 mg/kg BW and even more preferably 1 to 50 mg/kg BW by weight based on the total weight of the subject to whom the pharmaceutical composition will be administered.

According to the preferred embodiment, NFL-TBS₄₀₋₆₃ peptide, biologically active variants or pharmaceutically acceptable salts thereof is administered to a subject at dose concentration between 1 to 60 mg/kg BW /day, preferably between 1 to 40 mg/kg BW/day, more preferably between 1 to 20 mg/kg BW /day for 1-7 days and 1-10 cycles of administration. The daily dosage of the peptide as well as the duration of the treatment may be adapted for each subject in accordance with his/her health conditions and particularities.

According to the invention, the term **"subject"** means a human being, adult, juvenile or child.

According to the fourth aspect, the present invention relates to a method of treating cancer comprising a step of administering an effective amount of pharmaceutical composition according to the invention to a subject in need thereof.

The cancer, the route of administration and the dosage in this method are as those defined above.

The following examples and figures showing experimental results merely intend to illustrate the present invention.

### FIGURES

**Figure 1A****-D:** Image obtained by Transmission Electron Microscopy (TEM) showing the presence of fibrillar structures of NFL-TBS₄₀₋₆₃ peptide in four solutions as follows: **A.** NFL-TBS₄₀₋₆₃ (1 mg/mL) in water; **B.** NFL-TBS₄₀₋₆₃ (0.27 mg/mL) in water; **C.** NFL-TBS₄₀₋₆₃ in Formulation 24 (= 25 mM L-histidine, 2% Glycerol, 1% Glycine, 5% 2-HP-β-CD in WFI), and **D.** NFL-TBS₄₀₋₆₃ (1 mg/mL) in Formulation 1 (= 25 mM L-histidine, 1% Glycerol, 1% Glycine, 5% 2-HP-β-CD in WFI) . From **A** and **B,** it clearly appears that NFL-TBS₄₀₋₆₃ in water has a fibril structure. In **C,** the fibrils of NFL-TBS₄₀₋₆₃ instantly block a 0.2 µm filter. From **D** it appears that there are much less fibrils than in water (A and B) but fibrils are still present.
**Figure 2A****-B:** SEM images of NFL-TBS₄₀₋₆₃ peptide powder showing fibril-like structures in NFL-TBS₄₀₋₆₃ peptide powder (Lot AW18189D (figure **2A**) and Lot HF44004B (figure **2B**)).
**Figure 3****:** TEM image of a composition according to the invention containing 50 mM of L-alanine and 1 mg/mL NFL-TBS₄₀₋₆₃ peptide. The number and the size of the fibrils are significantly reduced, to the extent that virtually no fibrils were visible by TEM.

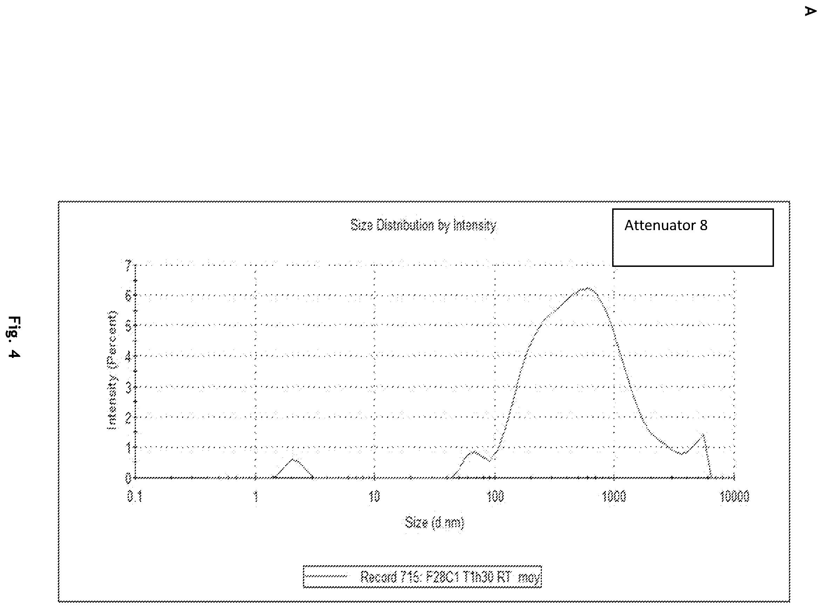

**Figure 4A****-C:** DLS graphs showing the effect of the heating of the formulation containing L-alanine at different temperatures before adding NFL-TBS₄₀₋₆₃ peptide followed by mixing for 1h30 at **A.** room temperature (RT); **B.** 30-35°C, and **C.** 40-45°C. The X-axis shows the size of the particles in nanometers (nm), and the y-axis shows the intensity (i.e. the amount of the particles present). In addition, the attenuator value also provides information on the amount of particles present in the sample, because it shows the level of light attenuation that the instrument needed to apply so as not to saturate its light detector: high attenuator numbers (e.g. ≥ 9) indicate that there were few particles in the sample that block/deviate the light. Low attenuator values (e.g. ≤ 8) indicate that there were substantially more particles in the sample. The best formulations had a high attenuator value, with low intensity peaks for particles > 50 nm.

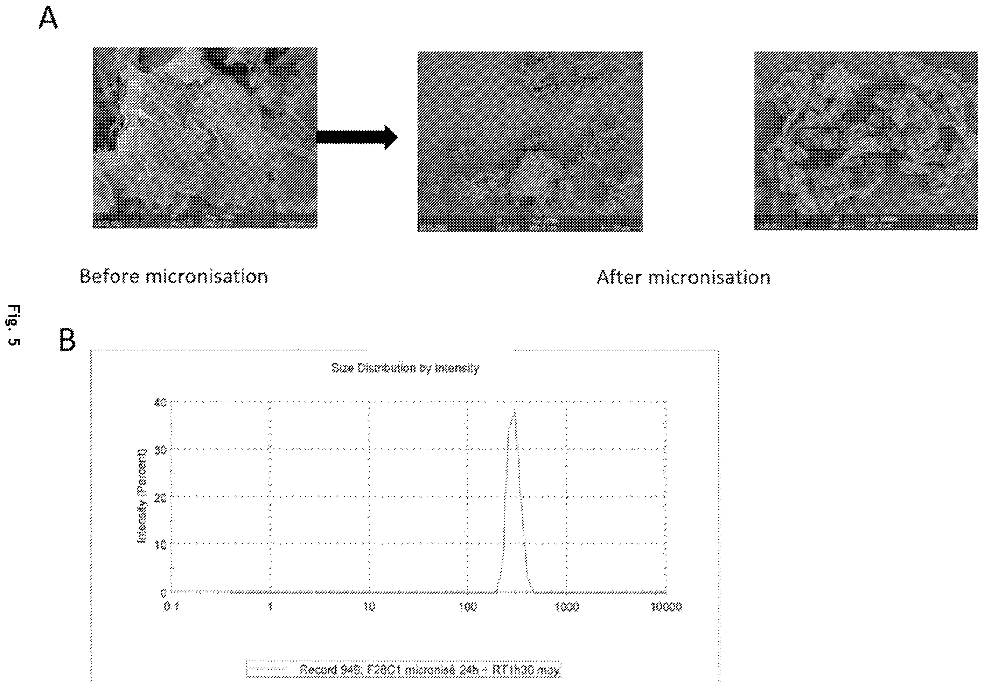

**Figure 5A****-B:** Micronisation test. **A.** SEM images of NFL-TBS₄₀₋₆₃ peptide powder before and after micronisation. **B.** DLS spectra of the dissolution of micronized NFL-TBS₄₀₋₆₃ peptide powder in F28C1 solution.

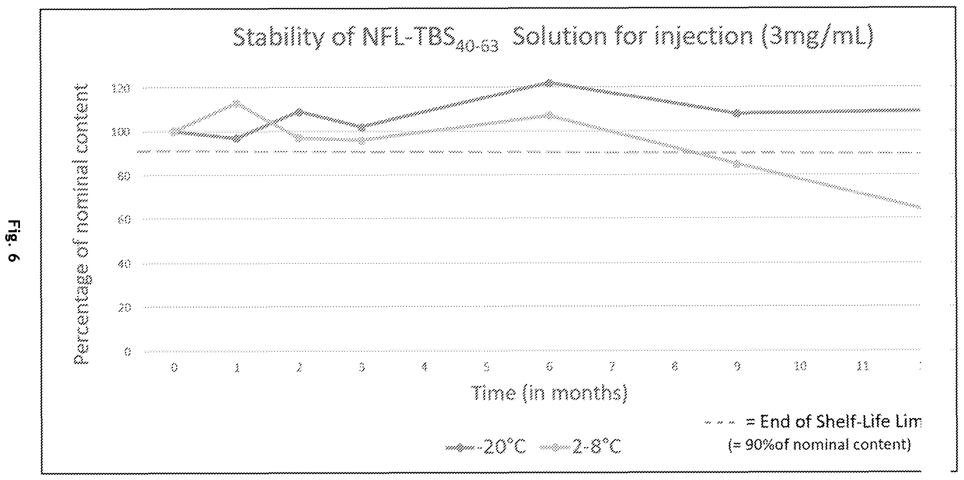

**Figure 6****:** Stability data for NFL-TBS₄₀₋₆₃ peptide solution for injection (3 mg/mL) at - 20°C and at 2-8°C, showing the variation of the content of the drug substance over time.

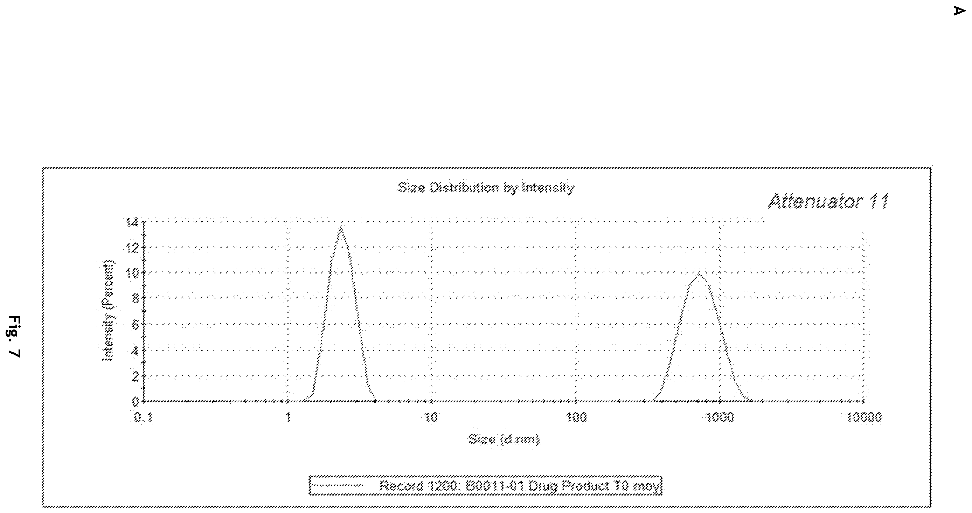

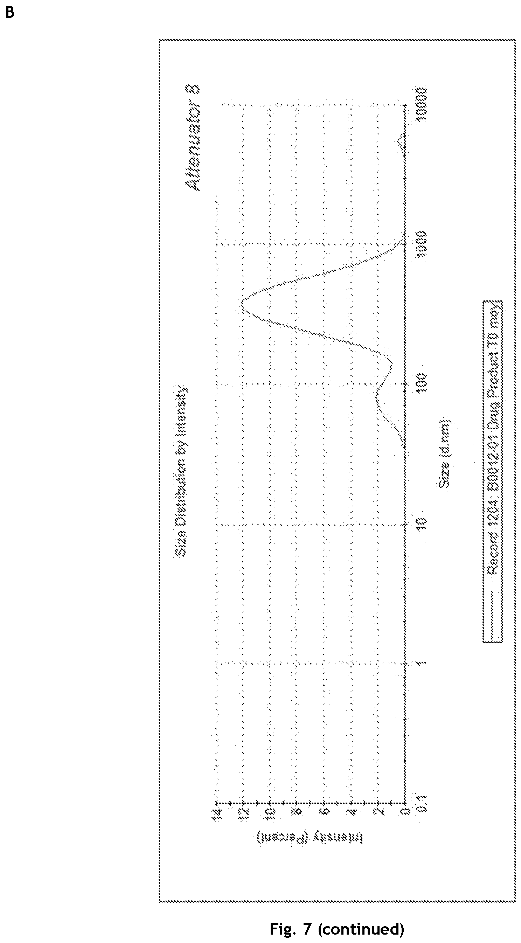

**Figure 7A****-C:** DLS spectra of the finished product batches in comparative example with three compositions: **A.** a placebo formulation of F28C1 without NFL-TBS₄₀₋₆₃ peptide; **B.** NFL-TBS₄₀₋₆₃ peptide (3 mg/mL) sterile, injectable solution in water with 5% glucose; **C.** NFL-TBS₄₀₋₆₃ peptide in F28C1 formulation

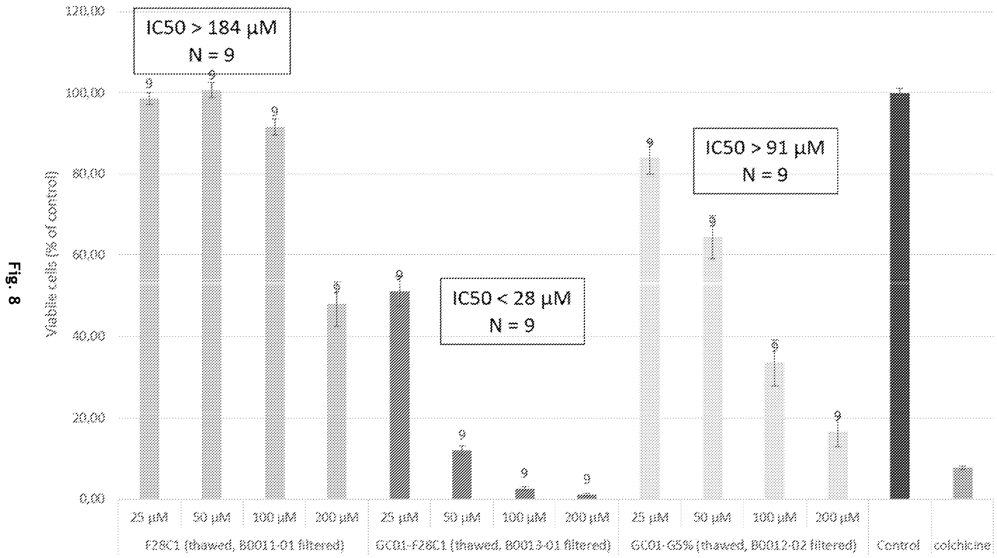

**Figure 8****:** Anti-tumor activity of NFL-TBS₄₀₋₆₃ peptide on rat glioblastoma cells F98. Cells were treated for 72h with increasing concentrations of peptide and then analysed with MTS assay (mean % of viable cells compared to the control (untreated cells) ± SEM. Each condition was analysed in triplicate and the analysis was performed only once (Negative control = untreated cells, with 100% survival rate; Positive control (colchicine) = cells treated with 1 µg/mL Colchicine; F28C1 = F28C1 solution, without NFL-TBS₄₀₋₆₃ peptide (formulation = 1% glycerol, 25 mM L-histidine, 50 mM L-alanine, and 10% 2-HP-β-CD in water for injections); GC01-F28C1 = 3 mg/mL NFL-TBS₄₀₋₆₃ peptide in F28C1 solution, and GC01-G5% = 3 mg/mL NFL-TBS₄₀₋₆₃ peptide in an aqueous solution of Glucose 5% in water for injections).

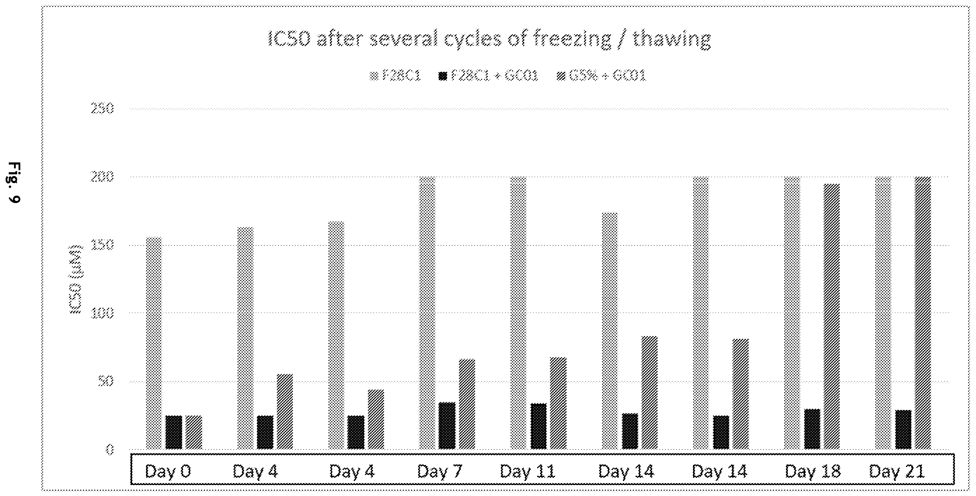

**Figure 9****:** Graphical representation of the IC50 in F98 cells over time treated with four compositions: F28C1 (without the peptide); GC01-F28C1; and GC01-G5%.

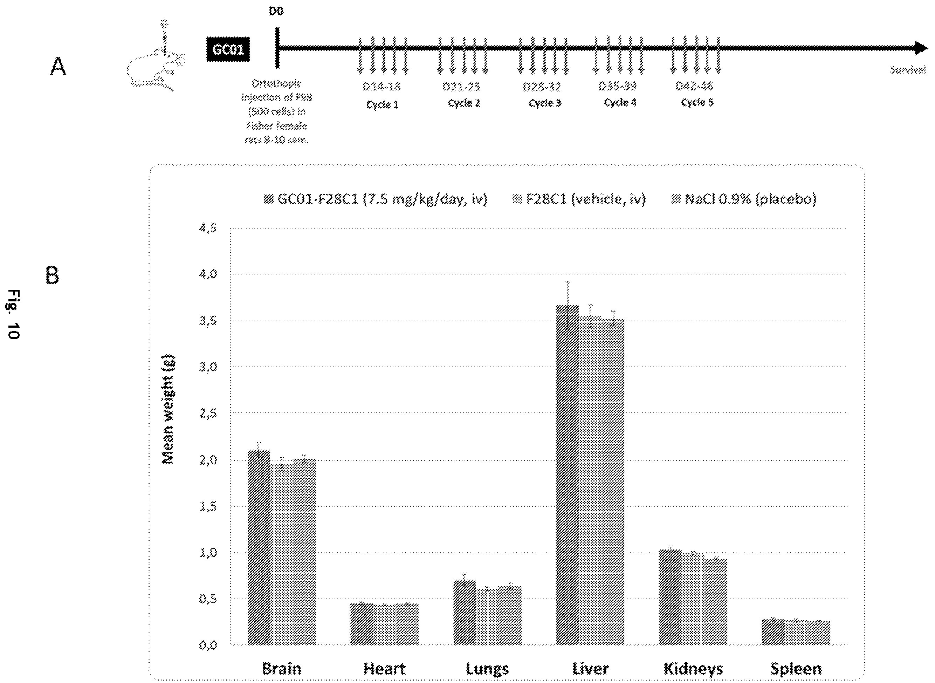

**Figure 10A-B** : A. Treatment scheme of *in vivo* assay in rats. **B.** Mean organ weight (g) at sacrifice in rats treated with F28C1 solution, without NFL-TBS₄₀₋₆₃ peptide, with GC01-F28C1 and with NaCl solutions.

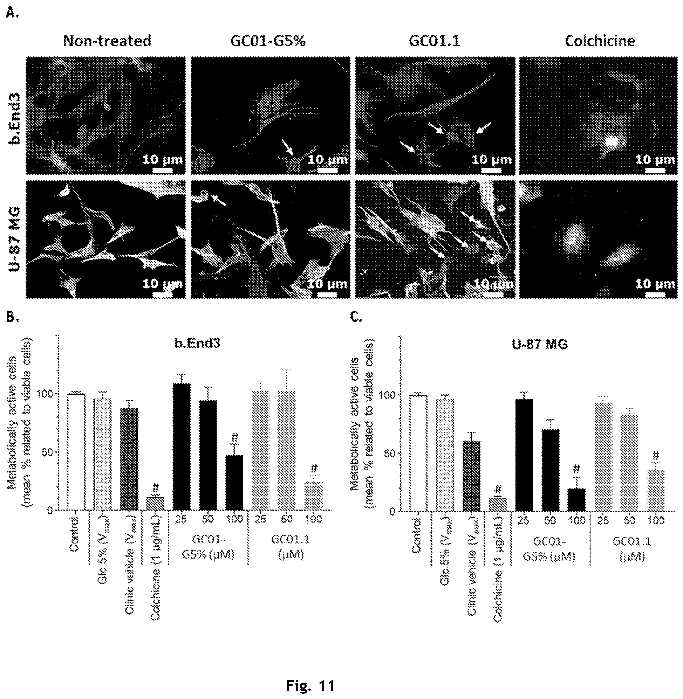

**Figure 11A-C** : *In vitro* effect of the peptide on cell viability of murine b.End3 endothelial cells and human U-87 MG GBM cells. **A.** b.End3 and U-87 MG cells were incubated with 100 µM of GC01-G5% (NFL-TBS₄₀₋₆₃ peptide (GC01) peptide partially solubilized with 5% glucose) or 100 µM of GC01.1 (NFL-TBS₄₀₋₆₃ peptide (GC01) fully solubilized in the formulation of the invention F28C1), or with 1 µg/mL of colchicine for 48 hours. Alpha-tubulin (in red) and BIII-tubulin (in green) were immunostained and nuclei were stained with DAPI (in blue). The white arrows show some cells whose microtubules are altered. Scale bar: 10 µm. Experiments were performed at least in n=3. **B.** Murine endothelial cells (b.End3) and **C.** Human GBM cells (U-87 MG) were treated for 72 hours at 37°C with increasing concentrations (25, 50, 100 and 200 µM) of GC01-G5% (NFL-TBS₄₀₋₆₃ peptide (GC01) partially solubilized with 5% glucose) or GC01.1 (NFL-TBS₄₀₋₆₃ peptide (GC01) fully solubilized in the formulation of the invention F28C1), or with 1 µg/mL of colchicine, or the 5% glucose solution and the Clinic vehicle. Cell viability (metabolically active cells) was evaluated by MTS assay. Experiments were performed at least in n=4-11. Data are represented as mean ± SEM. * and # represent significance (p < 0.05) compared with control (no treated cells, Kruskal-Wallis and a Dunn's post-hoc tests) and NFL (at the same concentration, Mann-Whitney test) respectively.

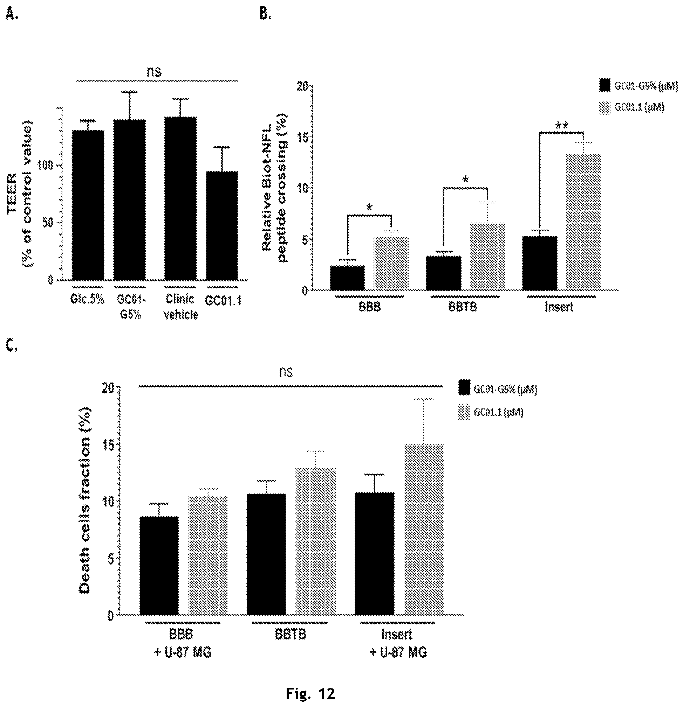

**Figure 12A-C** : Evaluation of GC01 formulations on *in vitro* BBB and BBTB models. **A.** The permeability of endothelial barrier of *in vitro* BBB model was assessed by measurement of the TEER values (Ohm x cm², % of control value) after 24 hours of treatment at 37° C with 100 µM of GC01-G5% (NFL-TBS₄₀₋₆₃ peptide (GC01) peptide partially solubilized with 5% glucose) or 100 µM of GC01.1 (NFL-TBS₄₀₋₆₃ peptide (GC01) fully solubilized in the formulation of the invention F28C1), or with the 5% glucose solution or the Clinic Vehicle. Experiments were performed at least in n=3. Data are represented as mean ± SEM. Statistical analysis by performing Kruskall-Wallis and Dunn's post-hoc tests. **B.** 100 µM of GC01-G5%, or GC01.1, were added in the luminal compartment of *in vitro* BBB and BBTB models for 24 hours at 37°C. The permeability of each model was assessed by LC-MS/MS method, to evaluate the quantity of GC01 peptide in abluminal compartment compared to the initial quantity in luminal compartment and was expressed as relative crossing material percentage (at the equilibrium between each compartment). Experiments were performed at least in n=3-4. Data are represented as mean ± SEM. Statistical analysis by performing Mann-Whitney test, * represents significance (p < 0.05). **C.** Quantification of calcein and PI staining was realized by flow cytometry on human GBM cells (U-87 MG) after passage of Biot-NFL peptide through the endothelial monolayer. After 24 hours of treatment, the endothelial cell monolayer was removed, and the U-87 MG cells were incubated at 37°C for an additional 48 hours. Results were expressed in percentage of death cells fraction. Experiments were performed at least in n=4-5 and 10,000 cells were measured for each experiment. Data are represented as mean ± SEM. Statistical analysis by performing Mann-Whitney test.

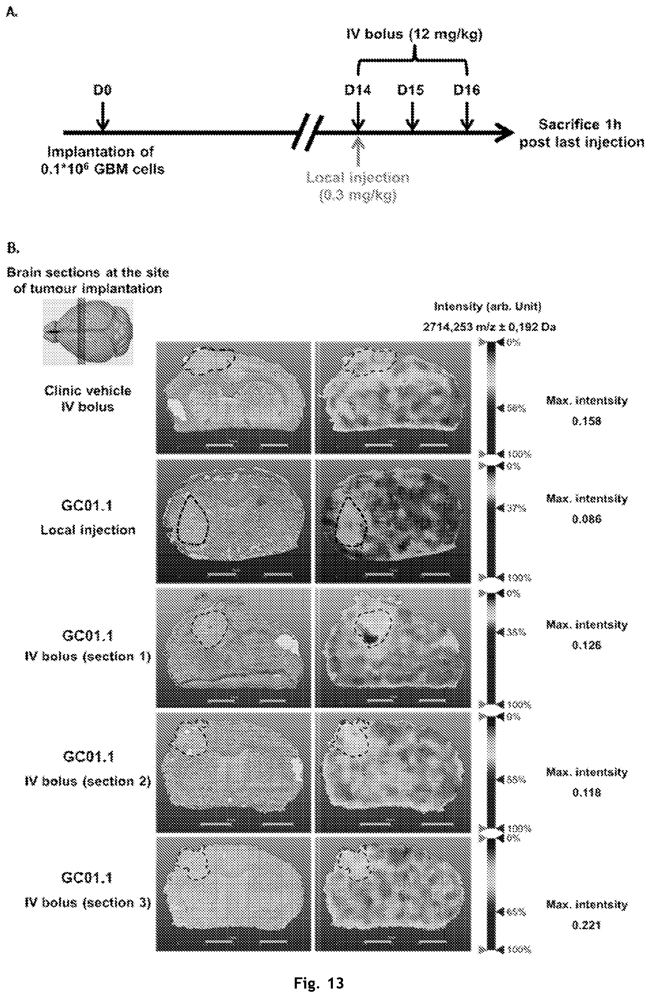

**Figure 13A-B** : *In vivo* imaging for the detection of specific accumulation of the biotinylated NFL peptide (GC01) in the tumor. **A.** Schematic representation of *in vivo* study timeline. The timeline of experiments began at day 0 with the implantation of 100,000 U-87 MG cells in nude mice by stereotaxic surgery. After 14 days post-implantation, clinic vehicle (F28C1) or GC01.1 were administered via an intravenous injection bolus at a dosage of 12 mg/kg for 3 days. One group of mice received a local injection of GC01.1 at a dosage of 0.3 mg/kg. Each group is composed of 3-4 mice. Mice were sacrificed 1 hour after the final injection. **B.** MALDI-TOF Imaging for evaluation of GC01 in GBM. These experiments were performed on a mouse brain sagittal section at the site of tumor. Data are shown using a rainbow scale (representing ion intensity scale) for best visualization. The tumor area is outlined in black Experiments were performed at least in n=1.

### EXAMPLES

### 1. NFL-TBS₄₀₋₆₃ peptide solubilization assays

Firstly, the inventors assessed several commonly used pharmaceutical excipients for dissolving NFL-TBS₄₀₋₆₃ peptide (said peptide is also called in the Examples below Biot-NFL or GCO1, particularly when the peptide bears a biotin molecule at the N-terminal and CONH₂ group (amide group) at the C-terminal and GC01.1, particularly when the peptide is in the formulation of the invention as for example in the formulation called F28C1) and obtaining a pharmaceutical injectable composition. Different methods (disclosed below) were used for observing the degree of solubilization of the peptide.

### 1.1. Materials:

Biotinylated NFL-TBS₄₀₋₆₃ peptide was synthetized and supplied by Polypeptide Laboratories France, Strasbourg France. Where possible, all pharmaceutical excipients were European Pharmacopoeia grade, and were supplied by Merck-Sigma.

### 1.2. Methods:

### Visual Dissolution Test

Initially, the solubility tests were performed by accurately weighing approximately 1 to 2 milligrams of NFL-TBS₄₀₋₆₃ peptide into a small colourless glass tube, and then sequentially adding 25µl portions of the test liquid (i.e. different kind of formulations tested for their ability to solubilize NFL-TBS₄₀₋₆₃ peptide), followed by vortex mixing for 2 minutes and visual assessment of dissolution of the NFL-TBS₄₀₋₆₃. The process was continued until either a volume was found at which the NFL-TBS₄₀₋₆₃ peptide powder appeared to be visually dissolved, or until the volume of liquid added reached 20mL without having found the point of dissolution.

Several test liquids were investigated, using combinations of practically all acceptable parenteral excipients, before surprisingly, finding that a specific concentration range of L-alanine was the key for optimal solubility of NFL-TBS₄₀₋₆₃ peptide.

### Clarity test - degree of opalescence

For preparations where the NFL-TBS₄₀₋₆₃ peptide appeared to be visually dissolved (following the visual dissolution test), the clarity (opalescence) of the preparation was assessed, via visual inspection after placing the colourless glass tube close to a white light source. The degree of opalescence was a very useful predictor of a given preparation's ability to pass or block a sterilizing filter.

### Filtration test

Several formulations (containing NFL-TBS₄₀₋₆₃ and various buffers, solubilizing and osmolarity adjusting agents) were tested for their aptitude to pass a 0.2µm filter. Initially, this was performed at small scale using a 13mm diameter Polyvinylidene fluoride (PVDF) filter connected to a syringe, which was used to test the passage of around 5mL of the test formulation. The ease of filtration (manual pressure required) was recorded. Potentially good formulations (where the peptide is correctly dissolved) passed the filter with little or no manual pressure (filtration grade 1), formulations with lower dissolution of the peptide required increasing force of manual pressure to pass through (grade 2), and poor formulations (wherein the peptide is poorly solubilized (dissolved)) either blocked the filter instantly or only allowed 1 or 2 drops of liquid to pass with difficulty before completely clogging up (grade 3).

For grade 1 formulations, further filtration tests were performed with higher volumes of the test formulation, and a deliberately small filter area size, e.g. a 25mm diameter with pores of 0.2µm with 100mL of the test formulation.

The aim of these tests was to eliminate formulations which contained non-dissolved NFL-TBS₄₀₋₆₃ peptide (eg. in fibrillar form) that would clog the sterilizing filter.

### Dynamic Light Scattering (DLS)

Dynamic Light Scattering (DLS) measurements were performed on the several formulations of grade 1 and 2 using a Malvern Nano-S instrument. Although the technique is not quantitative, it provided important information on the relative size of the objects (fibrils) present in the various formulations, throughout out the process of dissolution.

In particular, this technique helped the inventors to determine the optimum mixing times to achieve the smallest possible population of fibrils in the preparations prior to filtration.

### Transmission Electron Microscopy (TEM)

Transmission Electron Microscopy imaging of various formulations was performed using a Jeol JEM-1400 instrument. Sample preparation was as follows: one drop of the liquid to be examined was deposited onto a TEM gird and allowed to settle for ~1 minute. Excess liquid was then absorbed with blotting paper, and one drop of a 2% solution of uranyl acetate was deposited on the grid. After ~ 2 minutes the excess solution was absorbed with blotting paper, and the grid was rinsed twice, using with a drop of water each time. The grid was then dried, prior to examination in the TEM. This technique allowed visualization of the fibrils of NFL-TBS₄₀₋₆₃ peptide in the various formulations and in water.

### Scanning Electron Microscopy (SEM)

Scanning Electron Microscopy of NFL-TBS₄₀₋₆₃ peptide dry powder was performed using an Jeol 6301F instrument. In brief, NFL-TBS₄₀₋₆₃ peptide was lightly sprinkled onto double sided tape fixed to a SEM stub. Excess powder was shaken/tapped off, so as to leave a very thin layer of powder on the stub, which was then sputter coated with an ~10nm layer of platinum, prior to observation. This technique allows visualization of the fibrils in the dry NFL-TBS₄₀₋₆₃ peptide powder.

### High performance liquid chromatography (HPLC)

HPLC was used to quantify the content of NFL-TBS₄₀₋₆₃ peptide in some of the formulations of grade 1 after the dissolution process (before filtration) and after filtration. The HPLC analysis were performed on an Aglient 1260 Prime system with an Infinity Lab Poroshell 120 EC-C8 column.

HPLC was also used to assess the long-term stability of the optimal NFL-TBS₄₀₋₆₃ peptide parenteral solution formulation.

### 1.3. Results:

The images obtained by Transmission Electron Microscopy (TEM) show (**Figure 1**) the presence of fibrillar structures of NFL-TBS₄₀₋₆₃ peptide in four solutions as follows: NFL-TBS₄₀₋₆₃ in water (1mg/mL) (**Figure 1A**); NFL-TBS₄₀₋₆₃ in water (0.27mg/mL) (**Figure 1B**); NFL-TBS₄₀₋₆₃ (5mg/mL) in Formulation 24 (25mM L-histidine, 2% Glycerol, 1% Glycine, 5% 2-HP-β-CD in water ) (**Figure 1C**), and NFL-TBS₄₀₋₆₃ (1mg/mL) in Formulation 1 (= 25mM L-histidine, 1% Glycerol, 1% Glycine, 5% 2-HP-β-CD in water) (**Figure 1D**). In figures **1A** and **1B** it clearly appears that NFL-TBS₄₀₋₆₃ in water has a fibrillar structure. From Figure 1C it appears that with a sub-optimal formulation, 5mg/mL NFL-TBS₄₀₋₆₃ peptide forms a network of fibrils which instantly block a 0.2µm filter. Figure **1D** shows that when a formulation containing 25mM L-histidine, 1% Glycerol, 1% Glycine, 5% 2-HP-β-CD in water is used for dissolving 1mg/mL peptide, there are much less fibrils than at the same concentration in water (**Figure 1A**), but fibrils are still present and their presence is not compatible with sterilizing filtration and hence the solution cannot be used as a parenteral drug.

The inventors studied the NFL-TBS₄₀₋₆₃ peptide powder in order to see if the fibrils of the peptide are formed during the dissolution of the powder or if they are already present in the powder.

Images obtained by SEM of NFL-TBS₄₀₋₆₃ peptide powder (**Figures 2A****,B**) show fibril-like structures. It is clear that the fibrils are present in the dry powder of NFL-TBS₄₀₋₆₃ peptide and are "liberated" during the solubilization processes. Therefore, reducing the fibril number and size during the solubilization process appears to be the main challenge for formulating NFL-TBS₄₀₋₆₃ peptide as a solution for parenteral administration, rather than avoiding the gradual formation of fibrils over time and/or on agitation of the preparation.

As previously indicated, the inventors surprisingly found that the addition of specific concentrations of alanine (in particular L-alanine) allows to significantly reduce the fibrils in an aqueous composition in which NFL-TBS₄₀₋₆₃ peptide powder is solubilized. TEM images of a composition containing 50 mM of L-alanine and 1mg/mL NFL-TBS₄₀₋₆₃ peptide show that the number and the size of the fibrils of NFL-TBS₄₀₋₆₃ peptide is significantly reduced compared to similar compositions without alanine (as shown in **Figure 1A and D**).

### Assay on the effect of the concentration of L-alanine in F28C1 formulation

The effect of the concentration of L-alanine in formulation F28C1 has also been assessed. For this, five different concentrations of L-alanine (25mM, 50mM, 112mM, 225mM and 670mM) were assessed. The other components of the F28C1 formulations are: 25mM histidine buffer, 1% glycerol, 10% 2-HP-b-CD and water.

For assessing the effect of the concentration of L-alanine, five-formulations having different concentrations of L-alanine were filtered and the filtration ability of each formulation was evaluated by the above-described filtration test.

The following results were obtained:
- with a concentration of 50 mM L-alanine (~ 4.45 mg/mL L-alanine), a 1.5 mg/mL solution of NFL-TBS₄₀₋₆₃ peptide in F28C1 was very slightly opalescent, and 5mL of this solution containing 1.5mg/mL of NFL-TBS₄₀₋₆₃ peptide could be easily hand-filtered through a 13 mm diameter 0.2µm filter (filter grade 1 to 2);
- with 25 mM L-alanine (~ 2.3 mg/mL), a 1.5 mg/mL solution of NFL-TBS₄₀₋₆₃ peptide, the solution was slightly opalescent and filtration of 1mL through a 13 mm diameter 0.2µm filter became more difficult after the first 0.4mL had been passed (filter grade 2);
- with 112mM L-alanine (~ 10 mg/mL), a 1.5 mg/mL solution of NFL-TBS₄₀₋₆₃ peptide, the solution was slightly opalescent and filtration of 1mL through a 13 mm diameter 0.2µm filter became harder towards the end of the filtration (filter grade 2);
- with 225mM L-alanine (~ 20 mg/mL), a 1.5 mg/mL solution of NFL-TBS₄₀₋₆₃ peptide, the solution was slightly opalescent and filtration of 1mL through a 13 mm diameter 0.2µm filter became much harder towards the end of the filtration (filter grade 2 to 3), and
- with 670mM L-alanine (~ 60 mg/mL) a 0.5 mg/mL solution of NFL-TBS₄₀₋₆₃ peptide, the solution was slightly opalescent and filtration through a 13 mm diameter 0.2µm filter was very difficult, (filter grade 3).

It was estimated that the formulations having filter grade 1 or 2 may be used for manufacturing and injectable drug composition comprising NFL-TBS₄₀₋₆₃ peptide, when combined with a prefiltration prior to the sterilizing filtration.

From the above results, it became apparent that the working L-alanine concentration is in the range of 25 to 120 mM of L-alanine and the optimal concentration is around 50mM.

### 2. Optimization of the manufacturing method of the compositions comprising NFL-TBS_{40- 63} peptide and L-alanine

### ➢ Heating of the solution before adding TBS₄₀₋₆₃ peptide

The inventors assessed varying some parameters of the manufacturing method of the compositions of the invention in order to increase the solubility of the peptide and to eliminate the presence of fibrils. The final aim was to allow improvement of the ease of filtration of the composition.

Firstly, the inventors assessed the effect of the temperature on the solution before adding TBS₄₀₋₆₃ peptide. The tests have been performed at room temperature (RT), by heating at 30-35°C and at 40-45°C for 1.5h. The observation was made via DLS.

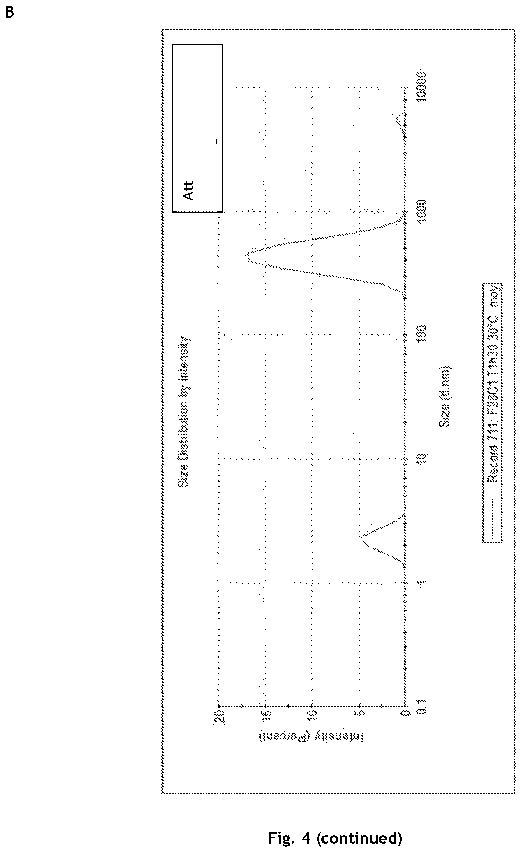

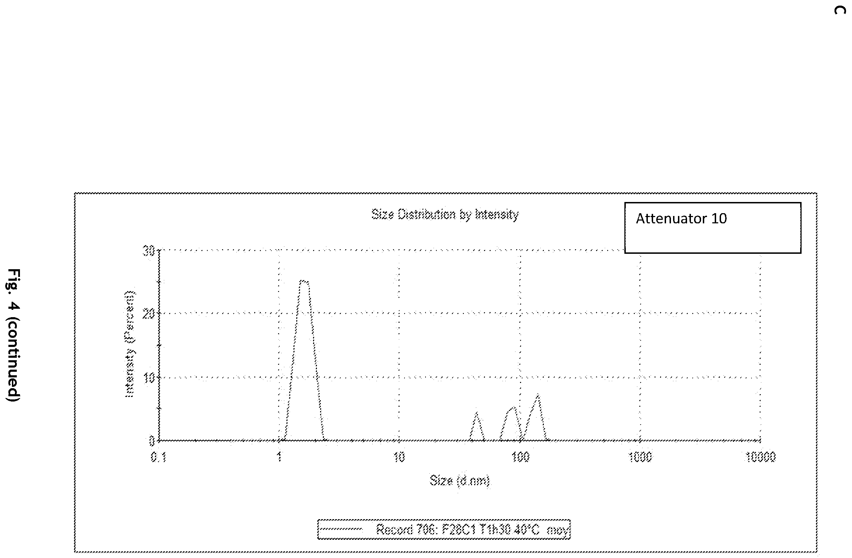

**Figure 4** shows DLS graphs, wherein the X-axis shows the size of the particles in nanometres (nm), and the y-axis shows the intensity (i.e. the amount of the particles present). The attenuator value also gives a measure of the quantity of particles present: the higher the attenuator value, the less particles present. The optimal manufacturing temperature leads to a high attenuator value (≥ 9), with low intensity peaks for particles > 50 nm. and indicates that the solution is proper for sterile filtration.

From **Figure 4** appears that the optimal temperature of heating of the solution F28C before adding the peptide is between 40 and 45°C (Figure 4C), wherein the attenuator value is 10.

The heating of the solution to this temperature allows to reduce the number and the size of the fibrils in NFL-TBS₄₀₋₆₃ peptide to the optimal degree, prior to filtration.

### ➢ Agitation during dissolution of the TBS₄₀₋₆₃ peptide powder

The inventors also found that the dissolution of the TBS₄₀₋₆₃ peptide is facilitated by agitation (eg. via magnetic or paddle stirring at around 300 to 600 rpm). An optimal time of stirring (1 to 2 hours) was found which minimizes the presence of fibrils, prior to filtration.

### ➢ Micronisation of TBS₄₀₋₆₃ peptide powder

The inventors performed micronisation tests at ambient temperature (about 20° C) by ball milling the NFL-TBS40-63 peptide for 48 hours. This was achieved by placing ~ 200 mg of NFL-TBS40-63 peptide powder in a 3.5cm diameter x 2cm high, round, metal container with a screw cap. One hundred (100) stainless steel ball bearings of 2mm diameter were added, and the metal container was closed and fixed onto a disk that can be rotated by a motor. The NFL-TBS40-63 peptide powder was ball-milled for 48h at approximately 100 - 120 rpm.

The results obtained by SEM observation before and after micronisation (**Figure 5A**) show that micronisation of NFL-TBS₄₀₋₆₃ peptide powder may be beneficial to "break up" some of the fibrils, and allow the dissolution process to be performed at ambient temperature.

Then, the dissolution of the micronized NFL-TBS₄₀₋₆₃ peptide powder in F28C1 formulation was tested with mixing for 1.5 hours at ambient temperature (20° C) at 5ml scale.

The DLS spectra (**Figure 5B**) prior to pre-filtration showed a single peak, with particles around 300 nm. This indicates that the majority of the micronized NFL-TBS₄₀₋₆₃ peptide powder was dissolved in the F28C1 formulation after 1.5 hours of mixing at ambient temperature.

The obtained composition with the dissolved peptide was submitted to pre-filtration through a 0.45µm filter. Then the pre-filtered composition was submitted to sterilizing filtration through a 0.2µm which was successful. This demonstrates that the micronisation of TBS₄₀₋₆₃ peptide powder before adding it to a formulation containing alanine (F28C1 formulation in this example) promotes the dissolution of the peptide at ambient temperature.

### HPLC analysis of the final formulation

With the optimized F28C1 formulation and production method described above (ie. heating to 40 to 45 °C and mixing for 1.5h prior to 0.45µm pre-filtration and 0.2µm sterilizing filtration), analysis by HPLC has demonstrated that the drug content in the NFL-TBS₄₀₋₆₃ solution for Injection (3mg/mL) is within ≥ 95 % of the nominal NFL-TBS₄₀₋₆₃ peptide content (3mg/mL). i.e. the composition and the manufacturing method are fit for purpose, i.e. obtaining a composition for parenteral administration.

HPLC has also been used to characterize the physico-chemical stability of the NFL-TBS₄₀₋₆₃ peptide (3mg/mL) in the optimal F28C1 formulation. The obtained data (**Figure 6**) indicate that the content of NFL-TBS₄₀₋₆₃ peptide remains above 90 % of the nominal content value for ≥ 12 months at -20°C, and for between 6 to 9 months at 2-8°C. This data demonstrate that the formulation has sufficient stability to make it industrially viable.

### 3. In vitro and in vivo assay

### In-vitro cytotoxicity (IC50) via MTS assay

The cytotoxicity of two formulations according to the invention (i.e. containing alanine) was assessed on some cell lines as described below.

### a. Cell culture

Cells were grown in a T75 flask (Sarstedt) until reaching 80-90% confluence, in a humid atmosphere at 37°C and 5% CO2 in media as per table 1.

**Table 1: Cell line culture material and method**

| Cell line | Species | Cancer type | Source, ref | Media |
|---|---|---|---|---|
| F98 | rat | Glioblastoma | ATCC, CRL-2397^{™}, | DMEM (Dulbecco's Modified Eagle's Mec (Gibco; Fisher Scientific) supplemented (Corning; Fisher Scientific), 1% NEAA Fisher Scientific), and antibiotics (strep Sigma) |
| U-87 MG | human | Glioblastoma | ECACC, 89081402 | DMEM (Dulbecco's Modified Eagle's Mec (Gibco; Fisher Scientific) supplemented (Corning; Fisher Scientific), 1% NEAA Fisher Scientific), and antibiotics (strep Sigma) |
| A172 | human | Glioblastoma | ECACC, 88062428 | DMEM (Dulbecco's Modified Eagle's Mec (Gibco; Fisher Scientific) supplemented (Corning; Fisher Scientific), 1% NEAA Fisher Scientific), and antibiotics (strep Sigma) |
| | | | | |
| b.End3 | murin | brain endothelioma | ECACC, 96091929 | DMEM + 2mM Glutamine + 5µM 2-Mercaptoethanol (2ME) + 1mM Sodium Pyruvate (NaP) + 1% Non Essential Amino Acids (NEAA) + 10% Foetal Bovine Serum (FBS). |
| hCMECd3 | human | Blood-Brain Barrier cell line | Millipore, Cat.#SCC066 | EndoGRO^{™}-MV Complete Media Kit (Cat. No. SCME004) supplemented with 1 ng/mL FGF-2 (Cat. No. GF003). |
| SH-SY5Y | human | Neuroblastoma | ECACC, 94030304 | Ham's F12:EMEM (EBSS) (1:1) + 2mM Glutamine + 1% Non Essential Amino Acids (NEAA) + 15% Foetal Bovine Serum (FBS). |
| A549 | human | Lung cancer | ATCC, CCL-185^{™} | |
| Daoy | human | Medulloblastoma | ATCC, HTB-186 | |

### b. Viability assay (MTS):

The cells were seeded in 96-well plates at 1,000 cells per well and incubated for 24 h at 37 ∘C and 5% CO2. Then, cells were treated for 72 h at 37 °C and 5% CO2 with increasing concentrations of the Biot- NFL-TBS₄₀₋₆₃ peptide (0, 25, 50, 100 or 200 µmol/L which is equal to 0, 0.0675, 0.135, 0.27 and 0.54 mg/ml respectively) solubilized in Glucose 5% solution or F28C1 formulation (after filtration), or with Colchicine (1 µg/mL), as positive control. Cell viability was measured using the MTS survival assay (ab197010; Abcam, Paris, France). After removal of the media, fresh media was added in each well to rinse cells. 100µL of fresh media supplemented with 20 µL of MTS reagent was added to each well for 2 h. The number of living cells is directly proportional to the absorbance measured by the amount of light absorbance at 490 nm in a SpectraMax M2 multi-scanning spectrophotometer (Molecular Devices, San Jose, California, USA).

The formulation F28C1 is a formulation according to the invention which further contains NFL-TBS₄₀₋₆₃ peptide, and the components shown in Table 2 below:

**Table 2: Content of formulation F28C1**

| **F28C1** |
|---|
| 50mM alanine |
| 25mM histidine buffer |
| 1% glycerol |
| 10% 2-HP-b-CD |
| q.s. water |

### In-vivo tolerance (rat)

The tolerance of NFL-TBS₄₀₋₆₃ peptide (3 mg/mL) in F28C1 formulation was tested in rats bearing F98 intracranial tumors. NFL-TBS₄₀₋₆₃ peptide in formulation F28C1, and F28C1 alone (vehicle), were administered via intravenous injection of a 7.5 mg/kg/day equivalent dose of NFL-TBS₄₀₋₆₃ peptide.

The administration was performed once-a-day for 5 consecutive days, for 5 weeks. The rats were frequently observed for any clinical signs of adverse reactions. At sacrifice, an autopsy was performed, organs were observed and weighted.

### Comparative in vitro assay

For comparative use in ***in-vitro*** and ***in vivo*** studies, NFL-TBS₄₀₋₆₃ peptide (3 mg/mL) sterile, injectable formulation was also prepared in a solution called G5%, of 5% glucose in water for injection. This formulation was substantially harder to produce, in that NFL-TBS₄₀₋₆₃ peptide was less well dissolved (even after heating for 1.5h at 40 to 45 °C), and the pre-filter and sterilizing filters tended to clog up quite quickly, indicating that some of the peptide was present as fibrils and was retained in the filter.

In addition, a placebo formulation of F28C1 without NFL-TBS₄₀₋₆₃ peptide was also produced.

These two compositions were compared to the composition comprising F28C1 solution and NFL-TBS₄₀₋₆₃ peptide (solution for injection (3 mg/ml)).

DLS spectra of the finished product batches (**Figure 7A****)** show that the attenuator of the DLS equipment was highest (attenuator 11) for the placebo formulation of F28C1 in which there was no NFL-TBS₄₀₋₆₃ peptide (i.e. the least amount of particles was found).

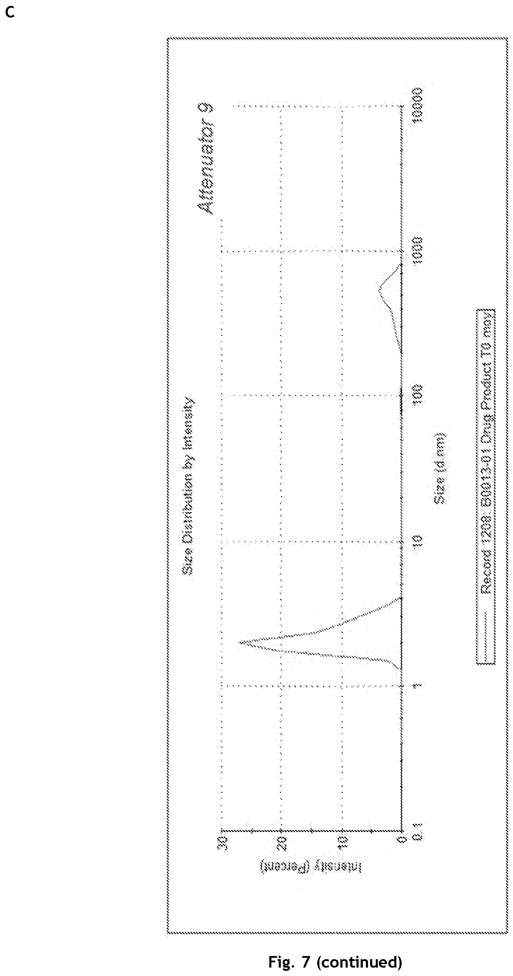

The attenuator value for the composition comprising NFL-TBS₄₀₋₆₃ peptide in F28C1 solution was 9, and it can be seen that a very low level of particles < 1000 nm in size was present (Figure 7C)

The attenuator value for the NFL-TBS₄₀₋₆₃ peptide in Glucose 5% was 8, and it can be seen that a higher level of particles in the 50 to 1000 nm in size were present, indicating that this formulation was not as good for solubilizing NFL-TBS₄₀₋₆₃ peptide which tended to re-form fibrils after the filtration steps.

These three compositions were then assessed for their anti-tumoral activity using the experimental protocol disclosed above. In addition, a negative control (untreated cells, with 100% survival rate) a positive control (cells treated with 1 µg/mL Colchicine) were used. The obtained results are as follows:

### a) MTS assays

It can be seen in **Figure 8** that the NFL-TBS₄₀₋₆₃ peptide (3 mg/ml) in formulation F28C1 is the most bioactive of the formulations with NFL-TBS₄₀₋₆₃ peptide (at 3 mg/mL).

At high concentration, it may be observed that the F28C1 solution (without NFL-TBS₄₀₋₆₃ peptide) has some limited anti-GBM activity. This may be explained essentially with the presence of 10% 2-HP-β-CD, which is known to have anti-proliferative activity against some cancers.

### b) IC50 values, and its stability over time after multiple free-thaw cycles

The solutions tested by MTS (above) were investigated to determine if their IC50 value changed after repeated freeze-thaw cycles. They were thawed and tested at different timepoints over 3 weeks. The solutions were re-frozen between tests.

The results are shown in the table 3 below, and graphically in **figure 9****:**

| **C50 (µM) in F98 cells** | | | |
|---|---|---|---|
| Timepoints | F28C1 (placebo) | F28C1 + GC01 | G5% + GC01 |
| T0 | 155,57 | < 25 | < 25 |
| T4 days | 162,98 | < 25 | 55,58 |
| T4 days | 166,95 | < 25 | 44,22 |
| T7 days | > 200 | 34,27 | 66,61 |
| T11 days | > 200 | 33,77 | 67,65 |
| T14 days | 174 | 26,26 | 83,68 |
| T14 days | > 200 | < 25 | 81,16 |
| T18 days | > 200 | 29,99 | 195,36 |
| T21 days | > 200 | 29,28 | > 200 |
| **Average** | **184,39** | **28,17** | **91,03** |
| StD | 6,37 | 1,27 | 21,04 |
| n | 3 | 3 | 3 |
| N | 9 | 9 | 9 |

From **Figure 9****,** It can be seen that NFL-TBS₄₀₋₆₃ peptide (3 mg/ml) in F28C1 solution is the only one of the three formulations tested, where the IC50 value remains stable (< 35 µM) throughout the subsequent freezing cycles over the test period. The IC50 value of the GC01-G5% solution increased with time, suggesting that fibrils re-formed in increasing amounts and that the non-soluble fibrils are less bioactive.

From these results appears that the composition comprising NFL-TBS₄₀₋₆₃ peptide in F28C1 solutions is superior to the other formulations with respect to maintenance of bioactivity during repeated freeze-thaw cycles.

### Comparative in vivo assay in rat

The tolerance of NFL-TBS₄₀₋₆₃ peptide (3 mg/mL) in F28C1 solution has been tested in rats (Female Fischer Rats), via intravenous injection of a 7.5 mg/kg BW/day dose of NFL-TBS₄₀₋₆₃ peptide (= 0.5 mL of the drug product per day for a rat of 200 g BW). The drug product was administered once-a-day for 5 consecutive days, for 5 weeks, starting 14 days after orthotopic transplantation of a F98 Rat Glioblastoma. The treatment scheme is shown in

### Figure 10A.

Two placebo groups (0.9% NaCl solution and the F28C1 buffer, without NFL-TBS₄₀₋₆₃ peptide) were also included in the test. The 0.9% NaCl solution was administered subcutaneously at a dose of 10 mL/kg at the same timepoints as the other formulations, whilst the F28C1 solution (without NFL-TBS₄₀₋₆₃ peptide), was administered intravenously with exactly the same dosage regime as the F28C1 formulation containing 3 mg/mL NFL-TBS₄₀₋₆₃ peptide (i.e. 0.5 mL per day for a 200 g rat). The rats were observed at least daily for any clinical signs of adverse reactions (Table 3).

**Table 4: Clinical signs of adverse reaction dues to the administration of tree different formulations**

| **Formulation** | **Route of administra tion** | **GC 01 Dose (mg/kg/day) and Volume/injection** | **Treatment schedule** | **Clinical signs or adverse reactions** | **Necropsy** | **Organ appearance and weight** |
|---|---|---|---|---|---|---|
| GC01-F28C1 | i.v. | 7.5 mg/kg/day | Days 1, 2, 3, 4, 5 / week, for 5 weeks | None, stable body weight | No unexpected findings | Normal |
| | | 0.5 mL / 200g BW | | | | |
| F28C1 buffer (without GC01 ) | i.v. | 0 mg/kg/day | Days 1, 2, 3, 4, 5 / week, for 5 weeks | None, stable body weight | No unexpected findings | Normal |
| | | 0.5 mL / 200g BW | | | | |
| NaCl solution | s.c. | 0 mg/kg/day | Days 1, 2, 3, 4, 5 / week, for 5 weeks | None, stable body weight | No unexpected findings | Normal |
| | | 2 mL / 200g BW | | | | |

From table 3 above it can be seen that the GC01-F28C1 formulation is well tolerated ***in-vivo,*** and no adverse clinical signs attributable to the drug product were observed.

At sacrifice, the following organs, brain, heart, lungs, liver, kidney and spleen, were observed and their weights were determined.

**Figure 10B** shows that no significant difference in the organ weights was observed compared to the two control formulations without NFL-TBS₄₀₋₆₃ peptide. This data further demonstrates that the GC01-F28C1 solutions is well tolerated *in-vivo.*

### Examples of formulations according to the invention

| **F28C1** |
|---|
| 3mg/mL GC01 peptide |
| 50mM alanine |
| 25mM histidine buffer |
| 1% glycerol |
| 10% 2-HP-b-CD |
| q.s. water |

| **Variant formulation #1** |
|---|
| 3mg/mL GC01 peptide |

| **100mM alanine** |
|---|
| 25mM histidine buffer |
| 1% glycerol |
| 10% 2-HP-b-CD |
| q.s. water |

| **Variant formulation #2** |
|---|
| 3mg/mL GC01 peptide |

| **100mM alanine** |
|---|
| 25mM histidine buffer |
| 1% glycerol |

| **7.5% 2-HP-b-CD** |
|---|
| q.s. water |

| **Variant formulation #3** |
|---|
| 3mg/mL GC01 peptide |
| 50mM alanine |
| 25mM histidine buffer |
| 1% glycerol |
| **10% sulfobuytlether- β-cyclodextrin** |
| q.s. water |

### 4. Ability of the biotinylated NFL-TBS₄₀₋₆₃ formulation (GC01.1) to cross the blood-brain barrier and target glioblastoma cells.

### 1. Materials and Methods

### 1.1 Peptides

The NFL-TBS₄₀₋₆₃ peptide coupled to the biotin (Biot-NFL) was dissolved at 3 mg/mL in Glucose 5% (from Corning^{™} glucose solution 45 % Media Tech^{™} - Fisher Scientific, Illkirch Cedex, France - diluted in MilliQ plus system - Millipore, Bilerica, USA) or in a solution according to the invention (i.e. containing alanine).

### 1.2 Cell culture and reagents

U-87MG human GBM cells, F98 rat GBM cells and b.End3 murine endothelial cells were obtained from European Collection of Animal Cell Cultures (Sigma-Aldrich; Saint-Louis, USA). They were cultured in Dulbecco's modified Eagle's medium high glucose (DMEM - Gibco; Dardilly, France) supplemented with 10 % of fetal bovine serum (Corning), 10 units of penicillin, 10 mg of streptomycin (Sigma-Aldrich; Saint-Louis, USA), and 1 % of non-essential amino acids (Lonza; Verviers, Belgium). Cell lines were cultured and maintained at 37 °C in a humidified atmosphere with 5 % CO₂.

### 1.3 Mitochondrial activity

Cells were seeded in 96-well plates at 1,000 per well for b.End3 murine endothelial cells and F98 rat GBM cells, and 5,000 cells and U-87 MG human GBM cells, and incubated for 24 hours at 37 ° C and 5 % CO₂. Then, the culture media was removed and cells were treated for 72 hours at 37 °C and 5 % CO₂ with increasing concentrations (25, 50, 100 and 200 µM) of GC01-G5% (NFL-TBS₄₀₋₆₃ peptide (GC01) partially solubilized with 5% glucose) or GC01.1 (NFL-TBS₄₀₋₆₃ peptide (GC01) fully solubilized in the formulation of the invention F28C1), or with 1 µg/mL of colchicine, or the 5% glucose solution and the Clinic vehicle, diluted in fresh media. Cell viability was measured using the MTS viability assay (ab197010 - Abcam; Paris, France). After rinsing the cells with 1X DPBS (Dulbecco's Phosphate Buffered Saline - Gibco; Dardilly, France), 20 µL of MTS reagent was added to each well for 2 hours. The number of living cells is directly proportional to the absorbance measured by the amount of light absorbance at 490 nm in a SpectraMax M2 multi-scanning spectrophotometer (Molecular Devices ; San Jose, California, USA).

### 1.4 Immunocytochemistry

To visualize the treatment internalization, 20,000 and 35,000 cells per well for b.End3 murine endothelial cells and U-87 MG human GBM cells, respectively, were seeded in 24-well plates containing one coverslip previously coated with poly(d)lysine 0.1 mg/mL per well and washed three times with 1X DPBS (Sigma-Aldrich; Saint-Louis, USA) and incubated for 24 hours at 37 ° C and 5 % CO₂. Then, the cells were incubated with 100 µM of GC01-G5% (NFL-TBS₄₀₋₆₃ peptide (GC01) peptide partially solubilized with 5% glucose) or 100 µM of GC01.1 (NFL-TBS₄₀₋₆₃ peptide (GC01) fully solubilized in the formulation of the invention F28C1), or with 1 µg/mL of colchicine for 48 hours at 37°C. At the end of the incubation time, cells were washed three times with 1X DPBS and fixed with 4 % paraformaldehyde (15714 - Delta microscopies; Mauressac, France) for 10 min at room temperature. Then, treated cells were permeabilized with 0.2 % triton X-100 (T9284 - Sigma-Aldrich; Saint-Louis, USA) for 10 min, and blocked with 5 % bovine serum albumin (A7030 - Sigma-Aldrich; Saint-Louis, USA) for 45 min at room temperature. Cells were co-incubated with a mouse anti-α tubulin (ab7750 - Abcam; Paris, France) and a rabbit anti-BIII tubulin antibodies at 1:250 (T2200 - Sigma-Aldrich; Saint-Louis, USA) overnight at 4 ° C. The primary antibody was revealed with antimouse Alexa Fluor 568 at 1:500 (A11004 - Fisher Scientific, Illkirch Cedex, France) and antirabbit Alexa Fluor 488 at 1:500 (A11008 - Fisher Scientific, Illkirch Cedex, France) for 1 hour at room temperature. Then, cells were incubated with 4',6-diaminido-2-phenylindole at 1 µM (DAPI, D9542 - Sigma-Aldrich; Paris, France) for 10 min at room temperature. For cells treated with liposomes, only nuclei were labelled with 1 µM of DAPI for 10 min at room temperature. All coverslips were mounted with the Prolong Gold Antifade reagent (P36930 - Fisher Scientific, Illkirch Cedex, France). Cells were observed with a confocal microscope (Leica TCS SP8 - Leica Biosystems; Nanterre, France).

### 1.5 Flow cytometry

The cellular uptake assessment of peptides was performed in U-87 MG, b.End3. Cells were seeded in 6-well plates at 400,000 cells per well and were incubated for 24 hours at 37 °C and 5% CO₂. Then, the cells were incubated with FAM-NFL with increasing concentrations of FAM-NFL peptide for 0.5, 1, 6 and 72 h at 37 ° C. After incubation, cells were washed with 1X DPBS and with free DMEM, and the cells were incubated with 1X Trypsin (Gibco; Dardilly, France) for 5 min. Cells were centrifuged at 250 G for 5 min, washed twice with 1X DPBS and counterstained with Propidium Iodide (50 µg/mL, P4867 - Sigma-Aldrich; Saint-Louis, USA). For the quenching of the autofluorescence, 0.1%Trypan Blue (Sigma-Aldrich; Saint-Louis, USA) was used before analysis on a BD FACSCantoTM II System (BD Biosciences; Paris, France) and using the FlowJo Software (BD Biosciences; Paris, France). 10,000 cells were measured for each condition.

### 1.6 Establishment of in vitro BBB (blood brain barrier) and BBTB (blood brain tight barrier) models

b.End3 cells (50,000 cells/cm² ) and U-87 MG cells (50,000 cells/cm² ) were seeded on the luminal side and the abluminal side of the insert, respectively. The following day, the two cell lines were cocultured to mimic the ***in vitro*** BBTB. Then, the insert was further cultured for 7 days to form a tight barrier. The confluency of the barrier was checked each day and the culture medium of the barrier was changed every 3 days.

After 24 hours of treatment with 100 µM of different treatments, the integrity of both co-culture endothelial barrier models was determined by measuring transendothelial electrical resistance (TEER) using EVOM2 (World Precision Instruments; USA). The TEER of endothelial barriers was also recorded throughout the experiment to monitor any possible cytotoxicity and loss of integrity after liposomes treatment.

### 1.7 In vitro transport across the BBB and BBTB models

To assess the percentage of peptide that passed through ***in vitro*** coculture models, the inventors used an LC-MS/MS method. Following this, after 7 days of culture to obtain BBB and BBTB models, the culture medium in the upper compartment was replaced with medium containing 100 µM of Biot-NFL peptide partially solubilized in glucose 5% or fully solubilized in the formulation of the invention (containing alanine). After 24 hours of treatment, 100 µL samples collected from wells were diluted with 275 µL of milliQ water to get a final volume of 375 µL. The samples were then filtered (ultrafiltration) throw Vivacon^{®} 500, 30,000 MWCO Hydrosart (Sartorius; Goettingen, Germany.), 10000 rpm, 15 min. Each sample was diluted 5 times, so the calculated concentrations of Biot-NFL were multiplied by 5 to get the right amount of compound in the samples. The chromatographic system used consisted of an ACQUITY PREMIER (Waters; Saint-Quentin-en-Yvelines, France) with autosampler set at 6 °C and column oven maintained at 30 °C. Peptide separation was performed on XBridge Premier Peptide BEH C18 column (130 Å, 2.5 µm, 2.1 mm × 100 mm, Waters; Saint-Quentin-en-Yvelines, France) with an elution gradient. Sample elution (10 µL injected) was performed at a flow rate of 0.5 mL/min with mobile phase A (water, FA 0.1%) and mobile phase B (acetonitrile, FA 0.1%) as follows: 0-4 min: 0-30% B; 4-4.5 min: 30% B; 4.5-5 min: 30-100% B; 5-7 min: 100% B; 7-7.5 min: 100-0% B; and 7.5-11 min: 0% B. Analyses were performed on a Xevo TQ-Cronos mass spectrometer (MS) (Waters; Saint-Quentin-en-Yvelines, France), in positive electrospray ionisation mode (ESI) and an acquisition mode in MRM (Multiple Reactions Monitoring). MS conditions were optimised with the capillary voltage at 3.4 kV, source temperature at 150 °C, desolvation temperature at 600 °C, and desolvation and cone source gas flow at 1000 L/Hr and 1 L/ Hr respectively. The voltage cone was optimized at 84,50 V. The quantification of GC01.1 (NFL-TBS₄₀₋₆₃ peptide in the formulation of the invention) corresponded to the sum of the peak obtained monitoring the transition of m/z 905.5 → 1162.9, 905.5 → 1075.9, 905.5 → 994.2 and 905.5 → 362.2 with respective collision energy of 20V, 26V, 26V and 40V.

### 1.8 In vitro anti-tumor efficacy of GC01 peptide using in BBTB models

After 24 hours of treatment, the endothelial cell monolayer was removed, and the U-87 MG cells were incubated at 37°C for an additional 48 hours. At this time, U-87 MG cells were collected as previously described in section 1.4. To evaluate cell viability were stained with 1 µM of Calcein green (Thermo Fisher Scientific; Massachusetts, USA) for 30 min at room temperature in the dark. Then re-suspended in Propidium Iodide and analyzed by flow cytometry. 10,000 cells were measured for each experiment.

### 1.9 Animal experiments

Seven-week-old female Balb/c nude mice (Janvier, France) were housed at the university animal facility (Service Commun d'Animalerie Hospitalo-Universitaire - Université d'Angers, France). Animals had free access to water and food throughout the experiment and their behavior and body weight were constantly monitored. All procedures involving animals, were conducted in accordance with protocols approved by ethical committee of the University of Angers and the regional ethics committee on animal experimentations. Furthermore, animal experiments were carried out in strict accordance with recommendations in the guidelines of the Code for Methods and Welfare Considerations in Behavioral research with Animals (European directive 2010/63/UE).

Mice were anesthetized by intraperitoneal injection of a mixture of ketamine 10% (80 mg/kg) and xylazine 2 % (10 mg/kg). Animals were then placed on a stereotaxic apparatus (Model 900 Small Animal Stereotaxic Instrument originally designed by David Kopf Instruments in 1963, Phymep, France) to be injected with 100,000 U-87 MG cells in 2 µL in the right frontal lobe with a Hamilton syringe. The injection coordinates were 2.1 mm lateral, 0.5 mm posterior from the bregma and 2.5 mm deep from the outer border of the cranium. 14 days post tumor graft, mice were treated with 12 mg/kg of GC01.1 injected in tail (I.V. bolus, once a day for 3 days) or 0.3 mg/kg of GC01.1 injected locally. Animals were sacrificed 1 hour after the last injection, and brains were removed and frozen in nitrogen vapors. The samples were stored at -80° before.

To detect the presence of GC01 (biot-NFL peptide) in the brain, a brain section at the site of tumor implantation was sliced to a thickness of 10 µm using a Leica CM1900 cryostat (Leica Microsystems GmbH; Wetzlar, Germany) at -20°C before then carried out and analyzed by MALDI-TOF imaging performed by the PROTISVALOR MaP platform (MSI; Marseilles, France). α-Cyano-4-hydroxycinnamic acid (CHCA) matrix was dissolved in ACN/H₂O (50:50, v/v) containing 0.1% trifluoroacetic acid (TFA) at the concentration of 10 mg/mL. The MALDI-TOF MS analysis was performed on a Bruker BIFLEX III mass spectrometer (Bruker Daltonics; Germany) equipped with a nitrogen laser (337 nm wavelength, 3 ns laser pulse duration) with reflection and in the positive ion mode. The MS data were acquired over a mass range of m/z 2714 Da with pixels of 100µm diameter but the capturing area is of 40µm per pixel. Data have been normalized by Root Mean Square and the image has been smoothed by reducing background noise. The maximum intensity of the images is in arbitrary units.

### 1.10 Statistical analysis

Data were presented as means ± SEM. Statistical differences were determined using unpaired Mann-Whitney test when comparing between two independent groups, and Kruskall-Wallis test followed by a Dunn's post-hoc test when comparing across three or more independent groups. p value < 0.05 was considered significant.

### 2. Results

### Antitumor activity of the biotinylated NFL peptide (GC01.1 and GCO1- G5%) and its ability to cross the BBB

First, immunocytochemistry was performed to assess the peptide effect on tubulin (**Figure 11A**). After 48 hours, cells treated with 100 µM of Biot-NFL peptides showed an alteration of tubulin. This effect is more pronounced on U-87 MG cells than on b.End3 cells as GBM cells express a higher level of BIII-tubulin, the main target of the peptide (Laurin et al., 2015 & 2017). Observations from the immunostaining appear to indicate a more significant alteration of tubulin in cells treated with GC01.1 compared to those treated with the Biot-NFL peptide partially solubilized in 5% glucose. Thus, these results highlight a similar cytotoxic activity between both formulations of Biot-NFL peptide, despite greater solubilization and effect on microtubules when solubilized in the clinical vehicle (F28C1). To complete this investigation, the *in vitro* effect of Biot-NFL peptides partially solubilized in Glucose 5% or fully solubilized in the formulation of the invention F28C1, were analyzed on the viability of murine b.End3 and human U-87 MG cells (**Figure 11B-C**). After exposure for 72 hours to various concentrations (0, 25, 50 or 100 µM), an MTS test was carried out. The cytotoxicity profiles were similar between the two cell lines with the different treatments, demonstrating that once in its Clinic vehicle, GC01 peptide retains its properties.

The permeability of the peptide through an *in vitro* BBB model was subsequently assessed. Initially, the integrity of the endothelial barrier was investigated following 24 hours of treatment with 100 µM of GC01.1, and GC01 in Glucose 5%. The TEER measurements, indicative of endothelial barrier integrity, revealed no significant differences between treated and untreated conditions, suggesting that these formulations did not affect it (**Figure 12A**).

To evaluate the capacity of the peptide to cross the endothelial barrier, 100 µM of GC01-G5% or GC01.1 were added to the luminal (upper) chamber for 24 hours. LC-MS/MS analysis was employed to determine the percentage of relative peptide that crossed the endothelial cell monolayer in the BBB and BBTB models. In order to determine the exact quantity of peptide present in the luminal compartment, the U-87 MG cells were removed prior to treatment. The passage of GC01.1 through the endothelial barrier models is significantly higher than that of GC01-G5%. In fact, when the treatments are deposited on the inserts without cells, we can observe a significant difference between the relative percentage of peptide crossing. In fact, only 10.4 +/- 1.2% of peptide passes when partially solubilized in 5% glucose while 26.7 +/- 2.6% when solubilized in the formulation of the invention (F28C1). These observations were also made for the ***in vitro*** models of BBB and BBTB. Only 4.8 ± 1.2% of GC01 partially solubilized in 5% glucose crossed the barrier compared to 10.4 ± 1.2% for GC01.1. An increase of 2 to 3% in the passage of peptide for both formulations was observed in the BBTB model compared to the BBB model. This suggests that the formulation of the invention containing alanine, by efficiently solubilising fibrils, improves its passage through the monolayer of endothelial cells (**Figure 12B**). Moreover, the passage of the peptide is greater in the model of BBTB than of BBB, suggesting that the microenvironment of GBM cells enhances the permeability of the endothelial cell monolayer, thus allowing a better passage of small molecules such as peptides. After 24 hours of treatment, the endothelial cell monolayer was removed, and the U-87 MG cells were incubated at 37°C for an additional 48 hours. At this time, U-87 MG cells were double labelled with calcein/PI and the fraction of dead cells was analyzed by flow cytometry (**Figure 12C**). It was noted that there was no significant difference between each condition, even though the percentage of dead cells tends to be higher in the presence of GC01. This might be due to the relatively low peptide concentration in the luminal compartment of each model. However, in coculture conditions, a percentage of dead cells ranging from 16.6 ± 5.4% to 30.1 ± 7.9% was still observed (for Transwell conditions + U-87 MG cells treated respectively with Biot-NFL and GC01) (**Figure 12C**). For the BBB and BBTB models, it was found that GC01.1 (GC01 fully solubilized in the formulation of the invention) has a greater impact on the viability of U-87 MG (19.2 ± 1.9% and 21.9 ± 4.6%, respectively) cells than when the Biot-NFL peptide was partially solubilized in 5% glucose (16.1 ± 2.0% and 17.5 ± 4.1%, respectively) (**Figure 12C**). Thus, the peptide solubilized in the formulation of the invention crosses the endothelial barrier better than the peptide solubilized in 5% glucose and maintains its ability to target GBM cells and induce cytotoxicity.

***In vivo*** experiments were conducted to confirm the capability of the peptide to target GBM when solubilized in the formulation of the invention. For this purpose, U-87 MG cells were implanted in the brains of immunodeficient BALB/c Nude mice. After 14 days, the mice were treated with the clinical vehicle F28C1 or GC01 in the formulation of the invention (GC01.1) at a dose of 12 mg/kg for 3 consecutive days. One additional group of mice received a local injection of GC01.1 at a dosage of 0.3 mg/kg. The multi-day treatment appeared to be well tolerated by the mice, with no observed weight loss. The mice were then euthanized 1 hour after the final injection (**Figure 13A**).

A brain section at the site of tumor implantation was then carried out and analyzed by MALDI-TOF imaging, as shown in **Figure 13B****.** The presence of the peptide accumulated at the tumor site is indicated by a rainbow color scale. A yellow, orange, or red coloration indicates the presence of the peptide in the observed area. Conversely, areas colored in green or blue represent places where the peptide is not detected. It's important to note that inevitable preparation artifacts can be observed, particularly at the edges of the brain slices. This was due to a folding of the section and/or a matrix effect. The local injection of the peptide at the site of the tumor cell implantation was performed as a positive control to ensure the reliability of the method for detecting the presence of the peptide in the brain. Areas colored in yellow, orange, or red at the tumor site were observed. These results demonstrated an accumulation of GC01 peptide in the tumor following intravenous injection of GC01 in the formulation of the invention, with the signal intensity varying depending on the section studied (**Figure 13B**). Consequently, this study demonstrates *in vivo* the ability of GC01 to cross the BBB and to target GBM cells, after intravenous injection in the formulation according to the invention.

### Comparative Example

The inventors tested the effects of several other amino-acids, particularly similar hydrophobic amino-acids such as L-valine, L-leucine, L-isoleucine, L-methionine as well as L-glutamine and L-proline. From the obtained results these amino-acids did not have any positive effect on the presence of fibrils in NFL-TBS₄₀₋₆₃ formulation. The results are shown in table 5 below:

| Amino Acid added into prototype formulation F28 * | Concentration of NFL-TBS₄₀₋₆₃ | Visual Solubility of NFL-TBS₄₀₋₆₃ (Vortex mixing @ 1500rpm) | Visual Clarity | Filtration 0.2µm (1mL; 13mmø) | Comments |
|---|---|---|---|---|---|
| L-Alanine 50 mM | 1 mg/mL | Dissolved in 2 mins | Clear | Very Easy (Level 1) | Promising formulation |
| L-Proline 200 mM | 1.5mg/mL | Dissolved in 8 mins | Quite clear | Medium resistance (Level 2/3) | Osmolality: 565mOsmol/kg Too high |
| L-Valine 100 mM | 1.5mg/mL | Dissolved in 6 mins | Quite clear | Medium resistance (Level 2/3) | - |
| L-Isoleucine 100 mM | 1.5 mg/mL | The peptide did not fully dissolve | n.t. | n.t | - |
| L-Leucine 50 mM | n.p. | n.p. | n. p. | n.p. | The L-Leucine did not dissolve in F28C. |

| | | | | | |
|---|---|---|---|---|---|
| n.p. = not tested * Prototype Formulation F28 = 25mM L-histidine, 2% glycerol, and 10% 2-HP-β-CD in purified water | | | | | |

### BIBLIOGRAPHICAL REFERENCES

[0] Golonov, A.P. et al (2004) A Simple Method for Improving Protein Solubility and Long-Term Stability. J. Am. Chem. Soc., 126, 8933-8939
[1] Berges, R. et al. (2012), A Tubulin Binding Peptide Targets Glioma Cells Disrupting Their Microtubules, Blocking Migration, and Inducing Apoptosis. Molecular Therapy vol. 20 no. 7, 1367-1377.
[2] Rivalin R. et al. (2014) The NFL-TBS.40-63 Anti-Glioblastoma Peptide Disrupts Microtubule and Mitochondrial Networks in the T98G Glioma Cell Line. PLOS ONE, June 2014 | Volume 9 | Issue 6 | e9847
[3] Lépinoux-Chambaud, C and Eyer, J. (2019) The NFL-TBS.40-63 peptide targets and kills glioblastoma stem cells derived from human patients and also targets nanocapsules into these cells. International Journal of Pharmaceutics 566, 218-228
[4] US Patent: US9023806_Peptide capable of altering tubulin polymerization and use thereof for inhibiting cell proliferation_2004
[5] US Patent: US9446092B2_2009_Use of a neurofilament peptide for the treatment of glioma
[6] Berges, R et al. (2012) Structure-Function Analysis of the Glioma Targeting NFL-TBS.40-63 Peptide Corresponding to the Tubulin- Binding Site on the Light Neurofilament Subunit. PLOS ONE, November 2012 | Volume 7 | Issue 11 | e49436
[7] Yokoo, M. et al. (2015) 2-Hydroxypropyl-β-Cyclodextrin Acts as a Novel Anticancer Agent. PLOS ONE | DOI:10.1371/journal.pone.0141946 page 1-20
[8] Irie, T. & Uekama, K. (1999) Cyclodextrins in peptide and protein delivery. Advanced Drug Delivery Reviews 36, 101-123
[9] Zapadka , K.L., et al. (2017) Factors affecting the physical stability (aggregation) of peptide therapeutics. Interface Focus 7:20170030. http: //dx.doi.org/10.1098/rsfs.2017.0030
[10] Falconier, R.J. et al. (2011) Stabilization of a monoclonal antibody during purification and formulation by addition of basic amino acid excipients. J Chem Technol Biotechnol 2011; 86: 942-948
[11] Maggio, E.T. (2010) Use of excipients to control aggregation in peptide and protein formulations. J. Excipients and Food Chem. 1 (2) 40-49.
[12] Hamada H., et al. (2009) Effect of Additives on Protein Aggregation. Current Pharmaceutical Biotechnology, 10, 400-407
[13] Bocquet A, et al. (2009) Neurosci 29: 11043-11054.
[14] Parker et al. (2017), Prevalence of bIII-tubulin (TUBB3) expression in human normal tissues and cancers Tumor Biology.
[15] Person et al. (2017), An Emerging Role for Tubulin Isotypes in Modulating Cancer Biology and Chemotherapy Resistance; International Journal of Molecular Sciences

## Claims

1. A pharmaceutical composition comprising NFL-TBS₄₀₋₆₃ peptide, biologically active variants or pharmaceutically acceptable salts thereof, an alanine and pharmaceutically acceptable excipients, wherein the alanine is at concentrations comprised between 25mM and 120 mM.

2. A pharmaceutical composition of claim 1, wherein the alanine is a L-alanine.

3. A pharmaceutical composition according to claim 1 or claim 2, wherein the pharmaceutically acceptable excipients are selected from the groups consisting of a buffer, an osmolarity adjusting agent and one or more solubilizing agent(s).

4. A pharmaceutical composition according to any one of the preceding claims, wherein:
- the buffer is selected from the group consisting of phosphate, citrate, L-histidine, acetate, lactic acid, tromethamine, gluconic acid, tartaric acid, succinate, malic, fumaric, α-ketoglutaric, an amino acid which is naturally positively charged at physiological pH, preferably the buffer is a L-histidine;
- the osmolarity adjusting agent is a polyol compound, preferably a glycerol, and
- the solubilizing agent is selected from the group consisting of a cyclodextrin, polypropylene glycol and polyethylene glycol (PEG).

5. A pharmaceutical composition according to claim 3 and claim 4, wherein the buffer is L-histidine, preferably at concentration comprised between 10-100 mM.

6. A pharmaceutical composition according to any one of the preceding claims which is an aqueous composition comprising:
- NFL-TBS₄₀₋₆₃ peptide, biologically active variants or pharmaceutically acceptable salts thereof at a concentration comprised between 1 and 20 mg/ml, preferably 3mg/mL
- an alanine at concentration comprised between 40 and 60 mM,
- L-histidine at concentration comprised between 10 and 100 mM,
- a glycerol, preferably at 0.5% to 5% of the total concentration of the composition and
- a cyclodextrin, preferably 2-Hydroxypropyl-β-cyclodextrin or sulfobuytlether- β-cyclodextrin at a concentration comprised between 0.5 and 50% of the total concentration of the composition.

7. A method for manufacturing a pharmaceutical composition according to any one of claims 1 to 6 comprising the following steps:
a) preparing a solution, preferably an aqueous solution, comprising a buffer, an osmolarity adjusting agent, one or more solubilizing agent(s) and alanine, preferably a L-alanine;
b) adding NFL-TBS₄₀₋₆₃ peptide, biologically active variants or pharmaceutically acceptable salts thereof to the solution prepared in step a);
c) pre-filtering the formulation obtained in step b) by using a filter with pore size compromised preferably between 0.3 and 1.0 µM, and
d) sterilizing the pre-filtered formulation of step c) by using a filter with pore size comprised preferably between 0.1 and 0.2 µM.

8. A method according to claim 7, wherein in step a):
- the buffer is selected from the group consisting of phosphate, citrate, L-histidine, acetate, lactic acid, tromethamine, gluconic acid, tartaric acid, succinate, malic, fumaric, α-ketoglutaric, an amino acid with is natural or positively charged at physiological pH, preferably L-histidine;
- the osmolarity adjusting agent is a polyol compound, preferably a glycerol, and
- the solubilizing agent is selected from the group consisting of a cyclodextrin, polypropylene glycol and polyethylene glycol (PEG).

9. A method according to claim 7 or claim 8, wherein in step b) NFL-TBS₄₀₋₆₃ peptide is a powder, preferably a micronized powder and/or a lyophilized powder, added at a concentration comprised between 2 and 5 mM, preferably 3mM.

10. A method according to any one of claims 7 to 9, wherein said method further comprises a step of heating the solution obtained in step a) and/or the formulation obtained in step b) at a temperature comprised between 20° C and 70° C, preferably between 40° C and 45° C.

11. A method according to any one of claims 7 to 10, wherein the addition of NFL-TBS₄₀₋₆₃ peptide, biologically active variants or pharmaceutically acceptable salts thereof in step b) is performed under agitation.

12. A pharmaceutical composition according to any one of claims 1 to 6 for use as medicament.

13. A pharmaceutical composition according to any one of claims 1 to 6 for use as a medicament for treating cancer.

14. A composition for use according to claim 13, wherein the cancer is a solid peripheral cancer or a cancer of the central nervous system.

15. A composition for use according to any one of claims 12 to 14 which is an injectable composition for parenteral administration.

## Patentansprüche

1. Pharmazeutische Zusammensetzung, die NFL-TBS₄₀₋₆₃-Peptid, biologisch aktive Varianten oder pharmazeutisch annehmbare Salze davon, ein Alanin und pharmazeutisch annehmbare Hilfsstoffe umfasst, wobei das Alanin in Konzentrationen zwischen 25 mM und 120 mM vorliegt.

2. Pharmazeutische Zusammensetzung nach Anspruch 1, wobei das Alanin ein L-Alanin ist.

3. Pharmazeutische Zusammensetzung nach Anspruch 1 oder Anspruch 2, wobei die pharmazeutisch annehmbaren Hilfsstoffe ausgewählt sind aus den Gruppen, die bestehen aus einem Puffer, einem Osmolaritätsregulator und einem oder mehreren Lösungsvermittlern.

4. Pharmazeutische Zusammensetzung nach einem der vorhergehenden Ansprüche, wobei:
- der Puffer ausgewählt ist aus der Gruppe, die besteht aus Phosphat, Citrat, L-Histidin, Acetat, Milchsäure, Tromethamin, Gluconsäure, Weinsäure, Succinat, Apfel-, Fumar-, α-Ketoglutar- und einer Aminosäure, die bei physiologischem pH-Wert natürlich positiv geladen ist, wobei der Puffer vorzugsweise ein L-Histidin ist;
- der Osmolaritätsregulator eine Polyol-Verbindung, vorzugsweise ein Glycerin, ist, und
- der Lösungsvermittler ausgewählt ist aus der Gruppe, die besteht aus einem Cyclodextrin, Polypropylenglycol und Polyethylenglycol (PEG).

5. Pharmazeutische Zusammensetzung nach Anspruch 3 und Anspruch 4, wobei der Puffer L-Histidin, vorzugsweise mit einer Konzentration zwischen 10 und 100 mM, ist.

6. Pharmazeutische Zusammensetzung nach einem der vorhergehenden Ansprüche, die eine wässrige Zusammensetzung ist, umfassend:
- NFL-TBS₄₀₋₆₃-Peptid, biologisch aktive Varianten oder pharmazeutisch annehmbare Salze davon mit einer Konzentration zwischen 1 und 20 mg/ml, vorzugsweise 3 mg/ml,
- ein Alanin mit einer Konzentration zwischen 40 und 60 mM,
- L-Histidin mit einer Konzentration zwischen 10 und 100 mM,
- ein Glycerin, vorzugsweise mit zwischen 0,5 % und 5 % der Gesamtkonzentration der Zusammensetzung, und
- ein Cyclodextrin, vorzugsweise 2-Hydroxypropyl-β-Cyclodextrin oder Sulfobutylether, β-Cyclodextrin, mit einer Konzentration zwischen 0,5 und 50 % der Gesamtkonzentration der Zusammensetzung.

7. Verfahren zur Herstellung einer pharmazeutischen Zusammensetzung nach einem der Ansprüche 1 bis 6, das die folgenden Schritte umfasst:
a) Herstellen einer Lösung, vorzugsweise einer wässrigen Lösung, die einen Puffer, einen Osmolaritätsregulator, einen oder mehrere Lösungsvermittler und Alanin, vorzugsweise ein L-Alanin, umfasst;
b) Beigeben von NFL-TBS₄₀₋₆₃-Peptid, biologisch aktiven Varianten oder pharmazeutisch annehmbaren Salzen davon, zu der in Schritt a) hergestellten Lösung;
c) Vorfiltern der in Schritt b) erhaltenen Formulierung unter Verwendung eines Filters mit einer Porengröße von vorzugsweise zwischen 0,3 und 1,0 µM, und
d) Sterilisieren der vorgefilterten Formulierung von Schritt c) unter Verwendung eines Filters mit einer Porengröße von vorzugsweise zwischen 0,1 und 0,2 µM.

8. Verfahren nach Anspruch 7, wobei im Schritt a):
- der Puffer ausgewählt wird aus der Gruppe, die besteht aus Phosphat, Citrat, L-Histidin, Acetat, Milchsäure, Tromethamin, Gluconsäure, Weinsäure, Succinat, Apfel-, Fumar-, α-Ketoglutar- und einer Aminosäure, die bei physiologischem pH-Wert natürlich positiv geladen ist, vorzugsweise L-Histidin;
- der Osmolaritätsregulator eine Polyol-Verbindung, vorzugsweise ein Glycerin, ist, und
- der Lösungsvermittler ausgewählt ist aus der Gruppe, die besteht aus einem Cyclodextrin, Polypropylenglycol und Polyethylenglycol (PEG).

9. Verfahren nach Anspruch 7 oder Anspruch 8, wobei im Schritt b) das NFL-TBS₄₀₋₆₃-Peptid ein Pulver, vorzugsweise ein mikronisiertes Pulver und/oder ein lyophilisiertes Pulver, ist, das mit einer Konzentration zwischen 2 und 5 mM, vorzugsweise 3 mM, beigegeben wird.

10. Verfahren nach einem der Ansprüche 7 bis 9, wobei das Verfahren ferner einen Schritt des Erwärmens der in Schritt a) erhaltenen Lösung und/oder der in Schritt b) erhaltenen Formulierung auf eine Temperatur zwischen 20°C und 70°C, vorzugsweise zwischen 40°C und 45°C, umfasst.

11. Verfahren nach einem der Ansprüche 7 bis 10, wobei das Beimengen von NFL-TBS₄₀₋₆₃-Peptid, biologisch aktiven Varianten oder pharmazeutisch annehmbaren Salzen davon in Schritt b) unter Rühren durchgeführt wird.

12. Pharmazeutische Zusammensetzung nach einem der Ansprüche 1 bis 6 zur Verwendung als ein Arzneimittel.

13. Pharmazeutische Zusammensetzung nach einem der Ansprüche 1 bis 6 zur Verwendung als ein Arzneimittel zur Behandlung von Krebs.

14. Zusammensetzung zur Verwendung nach Anspruch 13, wobei der Krebs ein solider peripherer Krebs oder ein Krebs des zentralen Nervensystems ist.

15. Zusammensetzung zur Verwendung nach einem der Ansprüche 12 bis 14, die eine injizierbare Zusammensetzung zur parenteralen Verabreichung ist.

## Revendications

1. Composition pharmaceutique comprenant le peptide NFL-TBS₄₀₋₆₃, des variants biologiquement actifs ou des sels pharmaceutiquement acceptables de celui-ci, une alanine et des excipients pharmaceutiquement acceptables, dans laquelle l'alanine est présente à des concentrations comprises entre 25 mM et 120 mM.

2. Composition pharmaceutique selon la revendication 1, dans laquelle l'alanine est une L-alanine.

3. Composition pharmaceutique selon la revendication 1 ou la revendication 2, dans laquelle les excipients pharmaceutiquement acceptables sont choisis dans les groupes constitués d'un tampon, d'un agent d'ajustement de l'osmolarité et d'un ou plusieurs agents solubilisants.

4. Composition pharmaceutique selon l'une quelconque des revendications précédentes, dans laquelle :
- le tampon est choisi dans le groupe constitué par le phosphate, le citrate, la L-histidine, l'acétate, l'acide lactique, la trométhamine, l'acide gluconique, l'acide tartrique, le succinate, l'acide malique, l'acide fumarique, l'acide α-cétoglutarique, un acide aminé qui est naturellement chargé positivement à un pH physiologique, de préférence le tampon est une L-histidine ;
- l'agent d'ajustement de l'osmolarité est un composé polyol, de préférence un glycérol, et
- l'agent solubilisant est choisi dans le groupe constitué d'une cyclodextrine, du polypropylène glycol et du polyéthylène glycol (PEG).

5. Composition pharmaceutique selon la revendication 3 ou la revendication 4, dans laquelle le tampon est la L-histidine, de préférence à une concentration comprise entre 10 et 100 mM.

6. Composition pharmaceutique selon l'une quelconque des revendications précédentes, qui est une composition aqueuse comprenant :
- le peptide NFL-TBS₄₀₋₆₃, des variants biologiquement actifs ou des sels pharmaceutiquement acceptables de celui-ci à une concentration comprise entre 1 et 20 mg/ml, de préférence 3 mg/ml
- une alanine à une concentration comprise entre 40 et 60 mM,
- de la L-histidine à une concentration comprise entre 10 et 100 mM,
- un glycérol, de préférence à une concentration comprise entre 0,5 % et 5 % de la concentration totale de la composition, et
- une cyclodextrine, de préférence la 2-hydroxypropyl-β-cyclodextrine ou la sulfobutyléther-β-cyclodextrine à une concentration comprise entre 0,5 et 50 % de la concentration totale de la composition.

7. Procédé de fabrication d'une composition pharmaceutique selon l'une quelconque des revendications 1 à 6, comprenant les étapes suivantes :
a) préparer une solution, de préférence une solution aqueuse, comprenant un tampon, un agent d'ajustement de l'osmolarité, un ou plusieurs agents solubilisants et de l'alanine, de préférence une L-alanine ;
b) ajouter le peptide NFL-TBS₄₀₋₆₃, des variants biologiquement actifs ou des sels pharmaceutiquement acceptables de celui-ci à la solution préparée à l'étape a) ;
c) préfiltrer la formulation obtenue à l'étape b) à l'aide d'un filtre dont la taille des pores est de préférence comprise entre 0,3 et 1,0 µM, et
d) stériliser la formulation préfiltrée de l'étape c) à l'aide d'un filtre dont la taille des pores est de préférence comprise entre 0,1 et 0,2 µM.

8. Procédé selon la revendication 7, dans lequel, à l'étape a) :
- le tampon est choisi dans le groupe constitué par le phosphate, le citrate, la L-histidine, l'acétate, l'acide lactique, la trométhamine, l'acide gluconique, l'acide tartrique, le succinate, l'acide malique, l'acide fumarique, l'acide α-cétoglutarique, un acide aminé naturel ou chargé positivement à un pH physiologique, de préférence la L-histidine ;
- l'agent d'ajustement de l'osmolarité est un composé polyol, de préférence un glycérol, et
- l'agent solubilisant est choisi parmi le groupe constitué d'une cyclodextrine, du polypropylène glycol et du polyéthylène glycol (PEG).

9. Procédé selon la revendication 7 ou la revendication 8, dans lequel, à l'étape b), le peptide NFL-TBS₄₀₋₆₃est une poudre, de préférence une poudre micronisée et/ou une poudre lyophilisée, ajoutée à une concentration comprise entre 2 et 5 mM, de préférence 3 mM.

10. Procédé selon l'une quelconque des revendications 7 à 9, dans lequel ledit procédé comprend en outre une étape où la solution obtenue à l'étape a) et/ou la formulation obtenue à l'étape b) est(sont) chauffée(s) à une température comprise entre 20 °C et 70 °C, de préférence entre 40 °C et 45 ° C.

11. Procédé selon l'une quelconque des revendications 7 à 10, dans lequel l'ajout du peptide NFL-TBS₄₀₋₆₃, de variants biologiquement actifs ou de sels pharmaceutiquement acceptables de celui-ci à l'étape b) est effectué sous agitation.

12. Composition pharmaceutique selon l'une quelconque des revendications 1 à 6, destinée à être utilisée comme médicament.

13. Composition pharmaceutique selon l'une quelconque des revendications 1 à 6, destinée à être utilisée comme médicament pour le traitement du cancer.

14. Composition destinée à être utilisée selon la revendication 13, dans laquelle le cancer est un cancer périphérique solide ou un cancer du système nerveux central.

15. Composition destinée à être utilisée selon l'une quelconque des revendications 12 à 14, qui est une composition injectable pour administration parentérale.
